(19) Europäisches Patentamt
European Patent Office
Office européen des brevets

(11) **EP 4 141 096 A1**

(12) **EUROPEAN PATENT APPLICATION**

(43) Date of publication:
**01.03.2023  Bulletin 2023/09**

(21) Application number: **21306144.3**

(22) Date of filing: **25.08.2021**

(51) International Patent Classification (IPC):
**C12M 1/12** (2006.01)   **B33Y 70/00** (2015.01)
**C12M 1/26** (2006.01)

(52) Cooperative Patent Classification (CPC):
**C12M 25/14; B33Y 10/00; B33Y 70/00;
B33Y 80/00; C12M 33/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **SARTORIUS STEDIM FMT SAS
13400 Aubagne (FR)**
• **UNIVERSITE CLAUDE BERNARD - LYON 1
69100 Villeurbanne (FR)**
• **Institut National des Sciences Appliquées de
Lyon
69621 Villeurbanne Cedex (FR)**

• **Ecole Supérieure de Chimie Physique
Electronique de Lyon
69616 Villeurbanne Cedex (FR)**
• **Le Centre National de la Recherche Scientifique
75794 Paris Cedex 16 (FR)**

(72) Inventors:
• **CHASTAGNIER, Laura
69100 Villeurbanne (FR)**
• **PETIOT, Emma
69100 Villeurbanne (FR)**

(74) Representative: **Mewburn Ellis LLP
Aurora Building
Counterslip
Bristol BS1 6BX (GB)**

(54)   **METHODS FOR PRODUCTION OF ENGINEERED CELLS**

(57)   Methods and systems for performing a bioproduction process are described, the bioproduction process comprising the production of a transformed cell population. The methods include the steps of culturing a cell population at least partially embedded in a hydrogel matrix forming a three-dimensional structure, the hydrogel matrix also comprising a transforming agent.

Fig. 1

EP 4 141 096 A1

## Description

Field of the Present Disclosure

The present disclosure relates to methods for the production of transformed cell populations and/or for the production of expression products by transformed cell populations, wherein the cells are transformed during the course of proliferation in a hydrogel matrix. Related methods, systems and products are described.

Background

Bioprocesses use biological systems to produce a specific biomaterial, for example a biomolecule with therapeutic effects. This process typically involves placing cells and/or microbes into a bioreactor with media containing nutrients under controlled atmospheric conditions. In the context of bioproduction, bioprocesses are typically performed as large-scale culture processes in a stirred tank bioreactor comprising liquid medium in which the cells are in suspension or cultured on microcarriers. More recently, adherent cell culture processes for bioproduction have been proposed in which the cells grow adhering to the surface of a scaffold, such as compacted fabric pieces or microporous carriers. These production types may be referred to as "fixed-bed bioreactor".

Many bioprocesses rely on obtaining transformed cells, for example to express a recombinant protein, produce a virus for a viral vaccine, etc. Transformation is a complex process, often requiring many manipulation steps. Further, cell transformation is often performed with low efficiency, can be transient, and is often poorly controlled. Various approaches have been proposed to enhance the efficiency of transformation of cells, including physical means (reviewed in Mellot et al., Ann Biomed Eng. 2013 March ; 41(3): 446-468), and the use of transfection reagents (e.g. lipofectamine, polyethylenimine, chitosan, modified alginates -see e.g. Padmanabhan & Smith, Pharm Dev Technol. 2002 Jan;7(1):97-101). However, any methods that does not involve an active step (e.g. repeated transformation during culture, use of physical means) still suffers from a poor control of the timing and location of the transformation. Methods that involve an active step are complex and represent failure points for the bioprocess, potentially resulting in large amounts of product failing to meet specification. Thus, the current approaches are limited both in terms of the complexity of the products that can be obtained, and the amount of product obtainable when expression of a product is transient.

Therefore, a need exists for improved systems and methods for the production of transformed cellular populations and products thereof, which do not suffer from all of the drawbacks of the prior art.

Summary

The present inventors have previously developed a bioink formulation for 3D printing of tissues, in particular skin substitutes (US 2019/0002836; Pourchet et al., Adv. Healthcare Mater. 2017, 1601101). For this purpose a solution comprising gelatin, alginate and fibrinogen together with fibroblasts was 3D printed into a thin solid structure and then keratinocytes were seeded onto the structure. This produced a skin substitute, with a strong accumulation of the fibroblasts at the interface between the printed object and the culture medium, forming a good substrate for the seeding of keratinocytes. These structures, including the support and the cell layers grown onto it, can be implanted directly as a skin substitute. More complex, porous structures such as a latticed cube were also evaluated for the production of larger amounts of skin substitutes without vascularisation, through improved culture medium perfusion (Pourchet et al., Bioprinting, Volume 20, December 2020, Pages e00114). The present inventors hypothesised that substantial cells growth could be achieved from cells at least partially embedded within a 3D hydrogel structure, i.e. that cells could grow to colonise the structure, and that this growth could be exploited to obtain transformation of the cells that enter into contact with a transforming agent also present in the hydrogel matrix, as a result of cell growth. The inventors therefore tested that transforming agents can be stably maintained in a hydrogel matrix, and can be taken up by cells that grow in said matrix thereby resulting in transformed cells. They showed that this method had numerous benefits including the ability to control the timing of transformation by controlling the respective location of the cells and transforming agents at the start of the culture (i.e. when the cells are "seeded"), the ability to transform new generations of cells as these grow to enter into contact with more transforming agent (without requiring additional active transformation steps to be included in the culture protocol), thereby resulting in long term transformation / expression of a product expressed only by the transformed cells, and improved transformation efficiency as the cells that enter into contact with the transforming agents are growing cells, which are more likely to take up a transforming agent.

According to a first aspect of the disclosure, there is provided a method for performing a bioproduction process comprising the production of a transformed cell population, the method including the step of culturing a cell population at least partially embedded in a hydrogel matrix forming a three-dimensional structure, wherein the hydrogel matrix also comprises a transforming agent, and wherein the growth of the cell population at least partially embedded in the hydrogel matrix results in the transformation of at least a part of the cell population by uptake of the transforming agent.

As mentioned above, the transformation of the cell population that is growing in the hydrogel ensures that growing cells are those that enter into contact with the transforming agent. Growing cells are believed to be more efficiently transformed than cells that are not actively growing. By contrast, in a process where the cells are brought into contact with a transforming agent in suspension, any contact between the transforming agent and the cells relies on mass transport phenomena in the liquid suspension (e.g. diffusion or sedimentation of the transfectant particles). Thus, there is not control over the proliferation status of the cells that enter into contact with the transforming agent. Further, the growth process within the hydrogel ensures that new generations of cells can reach further transforming agent, thereby resulting in sustained transformation (and hence sustained production of cellular products exclusively from transformed cells such as e.g. expression products encoded by nucleic acids in the transforming agent). By contrast, in a process where cells are transformed then cultured, the transformation process may have to be repeated a plurality of times to ensure sustained production of any cellular product that is exclusive to transformed cells. In addition, the timing of transformation can be effectively controlled, for example by controlling the amount and/or location of the cells and transforming agent, based on the knowledge of the growth rate of the cells in the hydrogel matrix. By contrast, in a process where the contact between the transforming agent and the cells relies on sedimentation/diffusion of the transforming agent or migration of the cells, any transformation typically occurs at the start of the incubation of the cells and transforming agent (e.g. in the first 24 hours), resulting in transient expression of any transgene within the first 1 to 3 days.

According to the present invention, the hydrogel matrix is not provided on a 3D structure, it is the hydrogel matrix itself that forms the 3D structure. Further, the cells are not seeded onto the hydrogel matrix, they are embedded in it. This is typically achieved by obtaining the hydrogel structure using a bioink composition comprising materials capable of forming a hydrogel and the cells. In other words, the hydrogel structure is typically formed from a solution comprising the cells, rather than from a composition that does not comprise cells, onto which cells are seeded / into which cells migrate.

Also described herein is a method for producing a transformed cellular population, the method comprising the steps of culturing a cell population at least partially embedded in a hydrogel matrix forming a three-dimensional structure, wherein the hydrogel matrix also comprises a transforming agent, and wherein the growth of the cell population at least partially embedded in the hydrogel matrix results in the transformation of at least a part of the cell population by uptake of the transforming agent.

The reference to a "transforming agent" encompasses the use of a single population of transforming agent as well as the use of a heterogeneous population of transforming agents or the use of a plurality of populations of transforming agents. For example, reference to a "transforming agent" may comprise the use of a plurality of populations of plasmids each having a different sequence.

The method of the first aspect may have any one or any combination of the following optional features.

The hydrogel structure may have been obtained by depositing one or more bioink compositions each comprising one or more biomaterials capable of forming a hydrogel and a cell population and/or a transforming agent in a controlled three-dimensional shape to obtain a cellularised structure, wherein at least one of the bioink compositions comprises a cell population and at least one of the bioink compositions comprises a transforming agent. The bioink composition may comprise a liquid culture medium. The bioink composition may have been obtained by mixing one or more solutions each comprising biomaterials capable of forming a hydrogel and/or cells in a liquid culture medium. The provision of the cell population and/or the transformation agent as part of a bioink comprising the biomaterial(s) that form the hydrogel matrix may advantageously enable a tight control of the cell seeding (or location of the transformation agent) such that this process is homogeneous throughout the structure. By contrast, in fixed bed bioreactors cell seeding is typically performed by flowing a solution of cells over the support, which typically results in a heterogenous cell seeding. This in turn may impact the cell density that is achievable in the process through local density limitation effects. By contrast, according to the present invention the cell seeding process may be much more homogeneous, thereby making it possible for the cell population to colonise the hydrogel structure with limited local density restrictions (until maximal cell density has been reached throughout the structure). Further, the provision of the cell population as part of a bioink comprising the biomaterial(s) that form the hydrogel matrix may advantageously enable the cells to be embedded in the hydrogel matrix at least at the start of the culture process, providing a protective structure for the cells to grow and colonise. The use of culture medium in the bioink composition advantageously results in improved growth rates and ultimately improved cell densities and transformation efficiencies in the cellularised structure. The culture medium use may comprise conditioning medium (i.e. culture medium in which the cells were cultured prior to formulation of the bioink).

The hydrogel structure may have been obtained by depositing a bioink composition comprising one or more biomaterials capable of forming a hydrogel, a cell population and a transforming agent. This may advantageously represent a simple process requiring only the formulation and deposition of a single bioink composition. This may further result in enhanced transformation efficiency, particularly at the beginning of the culture, and particularly when used in combination with additive manufacturing, as the mechanical stress associated with bioink preparation and printing (if used) may partially destabilise cell membranes thereby increasing the likelihood of early transformation.

The hydrogel structure may comprise a first region comprising the cell population at least partially embedded in the hydrogel matrix and a second region comprising the transforming agent in the hydrogel matrix. This may advantageously enable the control of both the timing and location of transformation. For example, different regions of the hydrogel structure may comprise different transforming agents or no transforming agent, resulting in a cellularised object that comprises a plurality of regions each comprising a cell population, wherein the cell populations in at least two of the regions differ by the presence or absence of a transforming agent. The first region may comprise a plurality of regions. The second region may comprise a plurality of regions. The hydrogel structure may have been obtained by depositing one or more first bioink composition(s) comprising one or more biomaterials capable of forming a hydrogel and a cell population to form one or more first regions of the hydrogel structure, and one or more second bioink composition(s) comprising one or more biomaterials capable of forming a hydrogel and a transforming agent to form one or more second regions of the hydrogel structure. The method may comprise depositing one or more layers of one or more first bioink composition(s) and depositing one or more layers of one or more second bioink composition(s). The method may comprise simultaneously depositing one or more first bioink composition(s) and one or more second bioink composition(s). A combination of two or more bioink compositions (such as a first and second bioink composition) may be simultaneously deposited using a nozzle that comprises different flow paths for the different bioink compositions. For example, a nozzle comprising a core and a sheath (or external) region may be used to deposit a bioink comprising cells and a bioink comprising a transforming agent (or vice-versa), respectively.

The transforming agent may comprise a non-viral vector, a viral vector or a virus. The non-viral vector may be a plasmid. The transforming agent and/or the hydrogel may comprise one or more additives. The one or more additives may be selected from a transfection reagent, and a reagent that binds or stabilise viral particles. Examples of reagents that bind or stabilise viral particles include viral receptors, sorbitol, trehalose, foetal bovine serum and sucrose (see e.g. Pastorino et al., 2015, PLoS ONE 10(4): e0118963). Examples of transfection reagents include cationic lipids (such as e.g. Lipofectamine from Invitrogen) and cationic polymers (such as e.g. polyethylenimine (PEI) and derivatives, polylysine and derivatives, chitosan and other amino containing sugars, or polyamidoamine dendrimers). For example, the transforming agent may comprise a transfection reagent selected from PEI and lipofectamine. Transfection reagents may be particularly useful in combination with non-viral vectors such as plasmids. As another example, the transforming agent or the hydrogel matrix may comprise a viral receptor or a sugar such as sucrose. These may be particularly useful in combination with viral vectors and viruses. Preferably, the transforming agent is obtained by incubating the non-viral vector, viral vector or virus with a reagent that binds or stabilises viral particles and/or a transfection reagent prior to including the transforming agent in the bioink composition. For example, the transforming agent may be obtained by incubating the active transforming agent (e.g. the non-viral vector, viral vector or virus) with the reagent that binds or stabilises viral particles and/or the transfection reagent prior to including the transformation agent in a solution comprising one or more biomaterials capable of forming a hydrogel. Thus, the method may comprise incubating the active transforming agent (e.g. the non-viral vector, viral vector or virus) with the reagent that binds or stabilises viral particles and/or the transfection reagent prior to including the transformation agent in a solution comprising one or more biomaterials capable of forming a hydrogel.

The hydrogel may have been obtained by consolidating one or more biomaterials capable of forming a hydrogel, wherein at least one of the biomaterials has been chemically modified to improve the stability and/or bioavailability of the transforming agent. The chemical modification may comprise the addition of positively charged groups. Examples of biomaterials that have been chemically modified to improve the stability and/or bioavailability of the transforming agent include cationised gelatin and cationised alignate. Thus, the method may also comprise using such chemically modified biomaterials to prepare a bioink from which the hydrogel structure is obtained, and/or obtaining such chemically modified biomaterials. For example, the method may comprise obtaining cationised gelatin from gelatin, obtaining cationised alginate from alginate, etc. Preferably, the transforming agent is incubated with a solution comprising a biomaterial that has been chemically modified to improve the stability and/or bioavailability of the transforming agent prior to formulation of the bioink composition (e.g. prior to mixing the solution comprising the transforming agent and the biomaterial that has been chemically modified to improve the stability and/or bioavailability of the transforming agent with one or more further solutions comprising further biomaterials capable of forming a hydrogel and/or cells). This may enable the modified biomaterial to interact with the transforming agent (e.g. by complexing it) to increase the effect of the presence of the modified biomaterial. Thus, the method may comprise incubating the transforming agent with a solution comprising a biomaterial that has been chemically modified to improve the stability and/or bioavailability of the transforming agent prior to formulation of the bioink composition. The method may comprise depositing a cationic bioink formulation comprising one or more biomaterials capable of forming a hydrogel and a cell population and/or a transforming agent, wherein at least one of the biomaterials is a cationic material. When the bioink composition comprises a reagent that binds viral particles and/or a biomaterial that has been chemically modified to improve the stability of viral particles by binding said viral particles, the method may comprise incubating the deposited object with one or more agents that cause the release of the viral particles. This may be performed, for example at the same time as consolidation of the deposited object. Alternatively, this may be performed at any point after consolidation of the deposited object. For example, the deposited object may be incubated with trypsin. This may cleave the bonds between the viral particles and/or the modified biomaterial.

The cell population may comprise or consist of cells from one or more cell lines, one or more populations of primary cells, or one or more production cell populations. The cell population may be a population of mammalian cells. The one or more production cell populations may be production cell lines selected from: an MDCK cell line, AGE.CR1™, PRE.C6, a VERO cell line, EB.14, EP.66™, HEK293, BHK21, a CHO cell line, NS0, Sp2, Sf9, SF21, MRC-5, WI-38, a CEF cell line, and a hybridoma cell line. The one or more production cell lines may be selected from a VERO cell line, a CHO cell line, an MDCK cell line, and HEK293. Production cells typically have been optimised for growth and/or productivity (e.g. protein expression rates) and hence have different characteristics and culture requirements from other cells such as primary cells. For example, production cells may have higher metabolic requirements (e.g. they may require more nutrients and/or oxygen in order to survive). Production cells typically grow as single cells or spheroids, whereas at least some primary cells and other cells have the ability to form organised structures such as organoids or tissues. Indeed, many production cell lines have been selected to be compatible with culture in suspension, and/or to grow as homogeneous population without directional/functional specialisation. The present inventors have discovered that production cells are particularly suitable for use in bioproduction processes according to the invention where the aim of the bioprocess is to produce a population of transformed cells or a cellular product that is a compound or component produced by transformed cells. Indeed, in such contexts the high growth rates and/or production rates of production cell lines can be optimally exploited in this context to obtain high cellular densities in culture. Nevertheless, the invention also finds uses in the context of transforming primary cells, such as e.g. cells obtained from a patient or animal model. In such cases, the present invention may advantageously enable the precise location of transformation of cells such that only a portion of the cells are transformed. This may be useful for example in the context of production of a tissue, or in any other context involving the production of a structure comprising cells having different characteristics (such as e.g. different functions, different cell types, disease and non-diseased cells, etc.).

The three-dimensional structure may be an object that has a 3D shape that is not merely a solid object having exclusively smooth surfaces (such as e.g. an object having exclusively flat surfaces and a certain constant thickness such as a parallelepiped). For example, a three-dimensional structure may be an object that has an external shape and an internal structure (such as e.g. one or more pores or cavities), and/or an external shape comprising one or more protrusions on at least one of its surfaces. In the context of the invention, such structures are typically 3D printed. This has many advantages as will be described further below, as it enables greater flexibility of design of the structure and enables the creation of pores / cavities of controlled shapes within the structure. As the cell population grows, the cells may at least partially or fully colonise the internal cavities / pores within the three-dimensional structure. Thus, the method may comprise maintaining the cell population until it reaches a desired density, wherein at the desired density the cells at least partially occupy the internal cavities / pores of the three dimensional structure. The three-dimensional structure may have one or more internal cavities. The three-dimensional structure may form a porous structure. The one or more internal cavities or pores may have a controlled size distribution. The one or more internal cavities or pores may have a size within a predetermined range of sizes. The three-dimensional structure may comprise a plurality of internal cavities arranged on a regular lattice. The one or more internal cavities or pores may form channels having a cross section between 400 and 800 $\mu m^2$, where these dimensions refer to the surface area of the cross section of pores forming a channel through the bioprinted object. The present inventors have found such dimensions to be particularly suitable for the high density culture of may cells including production cells. The three dimensional structure may not contain internal cavities. This is particularly suitable for three dimensional structures that are maintained in suspension in a liquid culture medium. Such three dimensional structures are typically smaller than three dimensional structures that are maintained on a support in a vessel. Embodiments comprising a three dimensional structure maintained on a support may advantageously be porous to enable an improved flow of culture medium throughout the structure, thereby ensuring that cells throughout the structure are adequately supplied with nutrients and gases.

The three-dimensional structure may have been obtained by additive manufacturing. The method may comprise obtaining a three-dimensional structure comprising a hydrogel matrix by additive manufacturing. The production of the three-dimensional structure by additive manufacturing is particularly advantageous as it enables the precise control of the geometry of the three-dimensional structure (including e.g. its porosity as well as the configuration of regions including cells vs. transforming agents), as well as the easy provision of a structure that has a different shape (whether overall or internal shape, such as e.g. porosity, total volume, etc.) for any use case, such as e.g. depending on the nature of the cell population, the composition of the hydrogel, the configuration of the bioreactor in which the structure is maintained (e.g. size, production scale, etc.), the desired timing and location of the transformation, etc. The method may comprise obtaining a three-dimensional structure by additive manufacturing comprises depositing a composition at a rate below 0.2 mm$^3$/s. Obtaining a three-dimensional structure by additive manufacturing may comprise depositing a composition as a filament, optionally wherein the filament has a diameter between 200 and 800 $\mu$m. The three-dimensional structure may comprise a plurality of cavities arranged on a regular lattice. The regular lattice may be a cube lattice. The use of a lattice may advantageously enable to produce a homogeneous and controllable flow of culture medium through the structure.

The three-dimensional structure may have dimensions such that the total (external) volume of the three-dimensional structure is in the order of cm$^3$ (e.g. 1 to 10 cm$^3$) or dm$^3$ (e.g. 1 to 10 dm$^3$). For example, the three-dimensional structure may have been obtained by depositing between 1 ml and 1l of bioink, between 1 ml and 500 ml of bioink or between 1 ml and 250 ml of bioink. Such three dimensional structures may be supported on a surface. Alternatively, the three-dimensional structure may have dimensions such that the total (external) volume of the three-dimensional structure is in the order of $\mu$m$^3$ (e.g. structures with an external diameter of between 50 $\mu$m and 500 $\mu$m, or with an external volume of 65.10$^3$ to 65.10$^6$ $\mu$m$^3$ or 0.065 mm$^3$) to mm$^3$ (e.g. 1 to 1000 mm$^3$). Such three dimensional structures may be cultured in suspension. Such three dimensional structures may be deposited as droplets rather than filaments. The three-dimensional structure may be deposited as a filament, such as e.g. by extrusion (e.g. pneumatic extrusion). The filament may have a substantially constant diameter between 200 and 800 $\mu$m. The present inventors have found such dimensions to be particularly advantageous in order to support the maintenance of cells at least partially embedded within the hydrogel matrix forming the filament. In particular, such dimensions resulted in particularly high cellular densities as they struck a good balance between structural integrity to support the growth of cells, providing sufficient surface area for the cells to grow on, and limiting the volume of matrix in which the cells can grow but do so typically to a lower extent. The composition may have been deposited at a printing rate below 0.2 mm$^3$/s. This may advantageously result in a low shear rate during printing, thereby increasing cell viability.

The method may comprise providing the bioink composition(s). The method may comprise depositing the bioink composition(s) in a controlled three-dimensional shape. The method may comprise consolidating the deposited bioink composition(s). Consolidating the bioink may comprise cross-linking one or more of the biomaterials capable of forming a hydrogel. The cross-linking conditions may be set to result in a degree of cross-linking that is compatible with the growth of the cells within the cross-linked matrix. Consolidating the bioink composition(s) may comprises exposing the deposited bioink composition(s) to one or more solutions that cross-link one or more of the biomaterials capable of forming a hydrogel. Providing the bioink composition may comprise incubating the bioink composition(s) at a predetermined temperature for a predetermined period of time such that the bioink composition reaches a viscosity compatible with printing without loss of cell viability. The provision of the cell population / transforming agent as part of a bioink comprising the biomaterial(s) that form the hydrogel matrix may advantageously enable a tight control of the cell seeding / location of transforming agent (and respective location of the cells and transforming agents, is these are provided in separate bioink compositions), such that the cells/transforming agent are homogeneously located throughout the structure or throughout the respective regions of structure where they are located. By contrast, in fixed bed bioreactors cell seeding is typically performed by flowing a solution of cells over the support, which typically results in a heterogenous cell seeding. This in turn may impact the cell density that is achievable in the process through local density limitation effects. By contrast, according to the present invention the cell seeding process (and location of transforming agent) may be much more homogeneous, thereby making it possible for the cell population to colonise the hydrogel structure with limited local density restrictions (until maximal cell density has been reached throughout the structure), as well as for the timing of contact between cells and transforming agents to be controlled by design rather than by manipulation (i..e through adding specific transformation steps). The step of providing the bioink composition may comprise a step of mixing a solution comprising cells in suspension and one or more solutions comprising biomaterials capable of forming a hydrogel. The step of providing the bioink composition may comprise a step of mixing a solution comprising transforming agents in suspension and one or more solutions comprising biomaterials capable of forming a hydrogel. The solution comprising transforming agents in suspension may also comprise one or more biomaterials capable of forming a hydrogel. The step of providing the bioink composition may comprise a step of mixing a solution comprising cells in suspension and one or more solutions comprising biomaterials capable of forming a hydrogel, wherein at least one of the one or more solutions comprising biomaterials capable of forming a hydrogel also comprises a transforming agent. The step of providing the bioink composition may comprise one or more steps selected from: preparing one or more solutions comprising biomaterials capable of forming a hydrogel (and optionally a transforming agent), amplifying a seed cell population, harvesting an amplified cell population, obtaining a solution comprising cells in suspension (such as e.g. by resuspending harvested cells), amplifying a transforming agent population, harvesting a transforming agent population, obtaining a solution comprising the transforming agent and one or more of the biomaterials capable of forming a hydrogel, and/or obtaining a solution comprising the transforming agent and a transformation additive. Consolidating the bioink composition may comprise exposing the deposited bioink composition to one or more solutions that cross-link one or more of the biomaterials capable of forming a hydrogel. For example, when the biomaterial capable of forming a hydrogel is alginate, consolidating the bioink composition may comprise exposing the deposited bioink composition to a solution that comprises calcium ions. As another example, when the biomaterial capable of forming a hydrogel is fibrinogen, consolidating the bioink composition may comprise exposing the deposited bioink composition to a solution that comprises thrombin. Advantageously, consolidating the bioink composition may comprise exposing the deposited bioink composition to a solution that comprises transglutaminase. The cross-linking of the biomaterials may include incubation with one or more cross-linking agents (e.g. calcium ions, thrombin, etc). The cross-linking conditions may include an incubation time, incubation temperature, and/or concentration(s) of one or more cross-linking agents. Thus, one or more of the cross-linking conditions may be set to those that result in a maximum cell density after a set time in culture, amongst a set of conditions tested. Thus, the method may further comprise culturing the cell population in a plurality of bioprocesses, wherein the plurality of bioprocesses differ in the consolidation conditions used to obtain the cellularised structure, and determining the consolidation conditions of the plurality of consolidation conditions that result in the highest cell density for the particular cell population and culture conditions after a predetermined culture time.

Providing the bioink composition may comprise incubating the bioink composition at a predetermined temperature for a predetermined period of time such that the rheological properties of the bioink composition are compatible with printing without loss of cell viability. Rheological properties that are compatible with printing and cell viability may be defined by defining a maximum static yield stress and/or maximum shear stress that does not result in substantial loss of viability, and a minimum static yield stress and/or maximum shear stress that results in a composition that can be deposited with a required level of shape fidelity. These may enable the skilled person to determine, for a composition having a particular set of rheological properties (such as e.g. as determined by obtaining a rheogram for the composition and comparing it to a reference rheogram), a range of temperatures that are compatible with printing and cell viability. For example, the bioink composition may be incubated for a set period of time at a temperature is below a temperature that would result in a loss of cell viability and that results in rheological properties that are compatible with printing and cell viability, such as e.g. a temperature at which the viscosity of the bioink composition is increased compared to a temperature at which the bioink composition was prepared. For example, the bioink composition may be incubated at a temperature between 21 and 28°C, such as e.g. approximately 21°C in order to increase the viscosity of the composition to enable the bioink composition to be printed into an object that maintains its shape, after preparation of the bioink composition at approximately 37°C (at which temperature the components of the bioink composition may be soluble and the composition may be fluid). It is advantageous for the viscosity of the bioink composition to be within a range that enables printing (i.e. such that a filament can be printed and maintain its shape) while not being so low as to increasing the shear rate experienced by the cells during printing to a level that would result in a loss of viability. The exemplary temperatures and compositions described herein are believed to have this effect, although alternative combinations of concentrations of components and temperature may be identified that would have the same effect. For example, increasing the concentration of gelatin may result in a bioink composition that would be incubated at a higher temperature to have the same viscosity. Further, the requirement for the bioink composition to maintain its shape when deposited may vary depending on the shape that is deposited (e.g. porous shapes may require higher shape fidelity than non-porous / solid objects) and the deposition configuration (e.g. supported shapes such as e.g. moulded shapes may not require high shape fidelity). The alginate may be low viscosity alginate. This may ensure that the viscosity of the bioink composition is not too high during printing. The method may further comprise determining the rheological properties of the bioink composition prior to deposition, for example by obtaining a rheogram of the bioink composition. The rheogram may then be compared with a reference rheogram to ensure that the bioink composition has desired rheological properties.

The hydrogel matrix may comprise alginate and fibrin. The hydrogel matrix may have been obtained by consolidation of a composition comprising alginate, fibrinogen and gelatin. The composition may comprise between 1.75% and 26% w/v of gelatin, between 0.15% and 4.2% w/v of alginate, and between 0.15% and 5.25% w/v of fibrinogen. Consolidation of the composition may comprise exposing the composition to a calcium salt and thrombin and/or wherein the composition is deposited in conditions enabling the gelatin in the composition to solidify thereby at least temporarily maintaining the 3D structure of the deposited composition. Hydrogel matrices comprising alginate and fibrin were found by the inventors to have a variety of properties that make them particularly suitable for the long term culture of production cells. For example, they are non-toxic to the cells, stable in culture medium for even long periods of time (both structurally and chemically), enable production cells to colonise the hydrogel structure (i.e. they do not hinder growth of such cells and do not require additional supplements in the culture medium to enable the cells to colonise the support, even for cells that are typically grown in suspension, in particular, the presence of fibrin promotes cell adhesion), and can be consolidated in conditions compatible with cell viability. Further, solutions comprising gelatin have the additional benefit that they can be 3D printed (or otherwise shaped) in conditions compatible with cell viability, prior to consolidation, for example by depositing or otherwise forming the object in conditions below the melting temperature of the gelatin (e.g. depositing the composition onto a cooled plate). The gelatin component can additionally be removed after consolidation, for example by incubating the object at a temperature above the melting temperature of the gelatin. Suitable compositions, preparations and printing processes are described in US 2019/002836 A1. However, according to the present invention (and contrary to what is disclosed in US 2019/002836 A1), it is preferable for the or each bioink composition to be prepared using culture medium as a buffer/solution for preparation of at least some of the components (preferably all of the components) of the bioink composition. Advantageously, such compositions are further compatible with observation of the cell population without separating the cells from the hydrogel matrix, such as e.g. during the course of the bioprocess. This may advantageously enable the monitoring of the culture, for example using optical and/or fluorescence microscopy. Thus, the method may comprise monitoring the bioprocess during the cell culture by examining the cell population at least partially embedded in the hydrogel matrix using optical and/or fluorescence microscopy. The culture medium may be free of calcium. In particular, if the bioink composition contains alginate, the culture media may be calcium free to prevent alginate chelation with calcium ions.

The cell population and hydrogel matrix structure may together form a cellularised structure and culturing the cell population at least partially embedded in a hydrogel matrix forming a three-dimensional structure comprises maintaining the cellularised structure in a liquid culture medium in a bioreactor. The cellularised structure may be completely immersed in the culture medium. The culture medium may be a chemically defined culture medium. The culture medium may be a serum free culture medium. The culture medium may be a protein-free culture medium. The culture medium may be free of additives used to protect the cells from hydrodynamic damage. The culture medium may be free of detergents. Conventional cell based production process often necessitate the use of additives to protect the cells from hydrodynamic stress. For example, additives such as pluronic (a Polyoxyethylene-polyoxypropylene block copolymer, a non-ionic detergent that is commonly used to protect cells from hydrodynamic damage) are frequently used in conventional stirred bioreactor bioprocesses. In the context of the present invention, such additives may not be necessary as the cells are protected by the hydrogel matrix and a flow can be used to provide the cells with the necessary gasses and nutrients instead of a rotary shaft and propellor as in common stirred bioreactor cultures.

Many conventional cell based production process require the addition of serum or other complex protein-containing mixtures, potentially of undefined chemical composition, in order to ensure the growth/fitness of the cells in the stress conditions of a stirred bioreactor, or to promote adhesion to a surface or carrier. In the context of the present invention, such additives may not be necessary are the cells are protected by the hydrogel matrix in which they are at least partially embedded.

The method may comprise maintaining the cell population until it reaches a predetermined cell density. The predetermined cell density may be at least $10^8$ cell/ml, at least $10^9$ or at least $10^{10}$ cell/ml. The cellular density may refer to the number of cells per unit of volume of bioink composition used to form the three-dimensional structure. The cellular density may be expressed as a number of cell per unit of volume (such as e.g. ml, $cm^3$, etc.). The volume of the three-dimensional structure may be equivalent to the volume of the composition used to form the three-dimensional structure in the case of a non-porous structure. In the case of a porous structure, the volume of the three-dimensional structure may be higher than the volume of the composition used to form the three-dimensional structure. For example, a three-dimensional structure that has a 50% porosity may comprise a total volume of composition that is half of the total volume of the three-dimensional structure. Thus, the number of cells per unit of volume of the three-dimensional structure may be half the cellular density expressed as a number of cells per unit of volume of composition used to form the three-dimensional structure. Further, the number of cells obtained may also be expressed relative to the volume of culture medium in the culture vessel, based on the cell density and the ratio between the volume of the composition used to form the three-dimensional structure (or the volume of the three dimensional structure and its porosity) and the volume of medium in the culture vessel. For example, using a known ratio between the volume of medium in the culture vessel and the volume of the composition used to form the three-dimensional structure (e.g. between 5 and 50), the number of cells may be expressed per unit of volume of the culture medium by dividing the cellular density by said ratio. Cellular densities of at least $10^8$ cell/ml, at least $10^9$ or at least $10^{10}$ cell/ml may therefore be equivalent to at least 0.2 to $0.002 \times 10^8$ cell/ml, at least 0.2 to $0.002 \times 10^9$ or at least 0.2 to $0.002 \times 10^{10}$ cell/ml of culture medium. The present inventors have discovered that high cell densities, such as e.g. $10^8$ cell/ml could be obtained within the context of the present invention at least partially because the cells can grow within the three-dimensional hydrogel structure (both within the hydrogel itself and in cavities/pores within the structure), such that the total cell density in the system is not limited by the presence of a support or carriers onto which the cells adhere but in which they cannot grow (resulting in an inherent density limitation caused by the available surface area and bulk of the support/carrier). Further, the method of the present invention is also not as limited as conventional stirred bioreactors in terms of cellular density as substantial flows of nutrients and gasses can be provided to the cells with a flow of liquid without damaging the cells as much as a stirring system that would be necessary to provide the same nutrients and gasses to a population of cells in a stirred bioreactor in which the cells are not protected by a hydrogel matrix in which they grow. The method may comprise maintaining the cell population until it reaches a predetermined size of cellular aggregates (e.g. spheroids). The predetermined size of cellular aggregates may depend on the cell type, and may for example range between approximately 20 $\mu$m in average diameter and approximately 100 $\mu$m or approximately 80 $\mu$m in average diameter. The present inventors have demonstrated that cellular aggregates with average diameters within these ranges could be obtained using a variety of commonly used production cell lines, using the process of the invention. The method may comprise maintaining the cell population until it reaches confluence, for example to form a tissue.

The method may comprise culturing the cell population for at least 7 days, at least 10 days, at least 15 days, at least 20 days, or up to 30 days. The growth of the cell population at least partially embedded in the hydrogel matrix may result in the transformation of at least a part of the cell population by uptake of the transforming agent after a predetermined amount of time in culture, after at least 3 days in culture, after at least 4 days in culture, after at least 5 days in culture, after at least 6 days in culture, from 3 days in culture to the end of the culture, or from 6 days in culture to the end of the culture. The present invention advantageously enables long term culture of the cells as well as long term expression of any product encoded by the transforming agent / long term transformation. Thus, the culturing of the cells may be performed over long time scales (e.g. at least 7 days, at least 10 days, at least 15 days, at least 20 days, or up to 30 days) without introducing additional transforming agent in the system. In other words, the method may comprise culturing the cells for such periods of time without adding transforming agent beyond the transforming agent already present in the hydrogel matrix. Further, the present invention enables the control of the transformation timing such that it only occurs after a predetermined amount of time in culture (such as e.g. 3 days or more ) and/or such that it occurs from said predetermined time to the end of the culture, By contrast, when using standard transformation protocols, the transformation typically occurs within the first 10 hours of contact with the transforming agent, with maximal expression of the transgene in the first 24 to 48 hours of co-culture with the transforming agent.

The method may comprise harvesting a cellular product wherein the cellular product comprises a cellular product exclusively or more effectively produced by transformed cells. The step of harvesting the cellular product may be performed one or more times during the culturing step and/or at the end of the culturing step. The cellular product may be selected from: a virus produced by transformed cells, an expression product of a transgene encoded by the transforming agent, a compound produced by the transformed cells, a tissue comprising transformed cells, a cellular population comprising transformed cells. The present invention advantageously enables the long term maintenance of cells with excellent viability as well as the long term production of any cellular product exclusively produced by transformed cells. This is partially due to the protective nature of the three-dimensional hydrogel structure, its biocompatibility, and the ability to renew the culture medium with limited damage to the cells, all of which ensure long term viability, and partially due to the transforming agent being also included in the hydrogel in which it is stable and able to enter into contact with new growing cells. This may be particularly useful for the production of viruses (e.g. for the production of viral vaccines) or of compounds exclusively or more effectively produced by transformed cells (including e.g. expression products of transgenes, compounds whose production is triggered or increased due to the presence of the transforming agent, etc.). Additionally, the present invention enables the localisation of the transforming agent, and the resulting transforming cells, in the hydrogel structure. This may be particularly advantageous for the production of tissues, models of pathology, drug discovery/testing systems, etc. where a plurality of cell populations with different characteristics are advantageously obtained at defined locations in the same hydrogel structure. For example, in the context of regenerative medicine, a tissue can be obtained comprising a plurality of transformed cell populations that secrete different growth factors, chemokines and/or differentiation factors, in order to obtain a heterogeneous tissue (e.g. a tissue comprising different cell types after exposure to said compounds secreted by transformed cells). As another example, in the context of drug discovery/testing, a tissue or structure comprising distinct populations of cells can be obtained, at least some of which are transformed so as to replicate a disease phenotype. Further, the present invention advantageously enables the easy harvesting of cellular products as any cellular product that is present in the culture medium can be harvested by drawing some of the culture medium (preferably with replacement) and any cellular product that is present in the cells (or is the cells themselves) can be harvested by extracting the cellularised structure and optionally separating the cells from the hydrogel matrix if desired (or directly using the cellularised structure as the cellular product of the bioprocess). Thus, the cellular product may comprise a biological compound or structure secreted or otherwise released by the cells in the cell culture medium. In such embodiments, the method may comprise harvesting the cellular product by at least partially removing the culture medium. In embodiments, harvesting the cellular product does not comprise a cell filtration step.

Thus, also described herein is a method of producing a virus, the method comprising performing the method of the present aspect, wherein the transforming agent comprises the virus or genetic material thereof. Similarly, also described herein is a method of producing a compound or complex the method comprising performing the method of the present aspect, wherein the transforming agent comprises one or more nucleic acids encoding for the compound or complex or encoding for a transgene the expression thereof causes the transformed cells to produce the compound or complex. Similarly, also described herein is a method of producing a tissue or population of cells, the method comprising performing the method of the present aspect, wherein the transforming agent comprises one or more nucleic acids encoding for a transgene the expression thereof causes at least some of the cells in the cell population to acquire a predetermined characteristic (e.g. cell type, disease phenotype, etc. ) The method may comprise exchanging at least part of the culture medium at least once during the bioproduction process. The method may comprise providing a flow of culture medium in the bioreactor. The method may comprise a step of washing the cellularised structure. The method may comprise maintaining the cellularised structure in a bioreactor comprising one or more inlets and one or more outlets to enable the addition of one or more solutions and the removal of culture medium from the bioreactor. The bioreactor may comprise a flow control device to control the flow of solution/medium in and out of the bioreactor. Because the cell population is at least partially embedded in the hydrogel structure, it is relatively easy within the context of the present invention to completely or partially exchange the culture medium in which the cellularised object is located. Indeed, conventional bioprocesses in which the cells are in suspension in the culture medium or on carriers in suspension in the culture medium require the provision of filtering devices in order to remove any culture medium for replacement. Such filtering devices have many issues, such as getting blocked, damaging the cells, resulting in the loss of at least some of the cells through incomplete filtration, etc. Thus, the provision of a system that does not require a filtration device for removal of culture medium from the bioreactor is particularly advantageous.

The culture medium may be exchanged fully at regular intervals, such as e.g. every 1, 2, 3, 4 or 5 days. This may be performed through a continuous process, where part of the medium is continuously being replaced, or through a semi-continuous process, where a part of the medium is removed and replaced at regular time intervals. The present inventors have found such medium replacement schemes to advantageously sustain cultures of multiple common production cell lines for long periods of time (such as e.g. up to 20 days or more).

The bioreactor may comprise one or more inlets and one or more outlets. These may be positioned relative to the cellularised object in order to optimise the flow of liquid through the cellularised structure. The flow of solution/medium in and out of the bioreactor may be controlled in order to optimise the flow of liquid through the cellularised structure. Optimising the flow of liquid through the cellularised structure may comprise maximising one or more criteria selected from the homogeneity of the flow through the cellularised structure, the cell viability, the cell density obtainable, etc. This may be performed using a flow modelling software. The provision of a flow of medium ensures that nutrients and gasses are provided to the cells, as well as preventing local accumulation of products and/or by-products of cell growth that may hinder cell growth. Further, a flow of culture medium through and/or around the hydrogel structure advantageously results in lower cell damage compared to stirring in a suspension system. This is believed to be at least in part because of the absence of a rotor and rotary motion, and because the cells are protected by the hydrogel matrix. According to any aspect of the invention, a bioink composition and/or culture medium may further comprise one or more additives, such as e.g. growth factors, nutrients, rheology additives, etc. One or more additives may be chosen specifically for the cell population.

The cellular product may comprise a biological compound or structure that is not released by the cells in the cell culture. The cellular product may comprise the cells themselves or a part thereof. In such embodiments, the cellular product may comprise the cells at least partially embedded in the hydrogel structure. For example, tissues, tissue models, drug discovery/testing models and the like may be obtained which comprise cells with desired characteristics (including transformed cells) at least partially embedded in the hydrogel structure. As a specific example, tissues for regenerative medicine may comprise at least some of the hydrogel structure, particularly where the hydrogel culture is a bioink as described herein and/or when the hydrogel is biocompatible. Alternatively, the cellular product may comprise purified cells. Thus, the method may comprise harvesting the cellular product by separating the cells from the hydrogel matrix, and/or washing the cellularised structure (or the separated cells). Separating the cells from the hydrogel matrix may be performed by mechanical separation and/or by dissolution of the hydrogel matrix. Harvesting the cellular product may in some embodiments not comprise a trypsination step. Advantageously, because the cells are at least partially embedded and/or contained within the hydrogel structure, the culture medium can be extracted without disrupting the cells, thereby removing the need for cell filtration in order to harvest the product. This makes it easier and more efficient to recover the product, as well as reducing the impact of product harvesting on cell growth and viability. Further, this reduces the problem of cell contamination that is often observed when filtration devices are used (e.g. in conventional stirred bioreactor processes). Additionally, such a harvesting / culture medium exchange process may be performed in a continuous or semi-continuous manner. Where the method comprises at least partially removing the culture medium, the method preferably comprises replacing at least part of the removed culture medium with fresh medium. Harvesting the cellular product may comprise extracting the cellular product from the culture medium. This may be performed using any process known in the art such as e.g. filtration, affinity purification, precipitation, centrifugation, etc. Advantageously, when the cellular product comprises the purified cells or parts thereof, growth at least partially embedded within the hydrogel matrix structure enables to reach high cell densities (typically higher than in e.g. stirred bioreactors or fixed bed bioreactors), thereby resulting in high product yield, without losing the benefits of easy recovery of the cells that is present in stirred bioreactors, and without the negative consequences of the use of a solid support where cells typically have to be separated from the support using treatments such as trypsinisation which can damage the cells. Further, because the cells are at least partially embedded and/or contained within the hydrogel structure, the entire cellularised structure can be easily washed to remove any contaminants, for example prior to harvesting the cells themselves or the entire cellularised structure, or to perform a complete change of medium. This is much more difficult to do in a traditional suspension culture, where washing of the cells often requires one or more cell separation steps (such as filtering or centrifugation), which are less efficient and more likely to damage the cells. While washing of the cells on their support may also be possible in a fixed bed reactor, this may cause damage to the cells. By contrast, according to the present invention, because the cells are at least partially embedded and/or contained within the hydrogel structure, any damage associated with washing is reduced. Mechanical separation of the cells from the hydrogel may comprise the use of a mechanical dissociator such as gentleMACS™. Dissolution of the hydrogel matrix may be performed using a chemical dissolution process, such as e.g. using EDTA or sodium citrate. Dissolution of the hydrogel matrix may be performed using an enzymatic process, such as using collagenase A, dispase, pepsin, papain, alginate lyase, trypsin, accutase, triplE, human platelet lyase, or matrix metalloproteinase(s). Dissolution of the hydrogel matrix may be performed by incubating the cellularised object in a solution comprising one or more chemical agents and/or one or more enzymes, then separating the cells from the supernatant for example by centrifugation or through density gradients. The cells may optionally be washed and/or resuspended, for example using a buffer such as PBS, after separation.

The cell population and hydrogel matrix structure may together form a cellularised structure and culturing the cell population at least partially embedded in a hydrogel matrix forming a three-dimensional structure comprises maintaining the cellularised structure in a liquid culture medium comprising at least a proportion of liquid medium in which the cell population has been cultured prior to formation of the cellularised structure. Instead or in addition to this, the bioink formulation used to obtain the cellularised structure may comprise at least a portion of liquid medium in which the cell population has been cultured prior to formation of the cellularised structure. The present inventors have discovered that the use of the culture medium in which the cells were culture prior to being included in the hydrogel matrix (also referred to herein as "conditioning medium") either as part of the bioink formulation or as part of the culture medium in which the cellularised object is initially cultured advantageously improves the growth rate and/or cell density achievable using the methods of the disclosure. Where the culture medium is replaced at least once, wholly, partially or continuously during the culturing, the proportion of said conditioning medium may decrease (or be equal to 0) after such replacement. However, the inventors believe that a benefit is still achieved provided that the cell culture in the cellularised structure is initiated in a culture medium comprising conditioning medium. The exact proportion of conditioning medium in new medium is not believed to be crucial. For example, this proportion may be adapted depending on the specific requirements of a bioprocess and/or cell line.

The method may further comprise providing a cellularised structure comprising the cell population and hydrogel matrix prior to the culturing, wherein the cell density in the cellularised structure is between $1.10^5$ and $1.10^7$ cell / ml of hydrogel matrix or of the composition from which the hydrogel matrix is formed. The present inventors have discovered that these cell densities (also referred to herein as "seeding density") advantageously result in higher growth rates / higher final cell densities when using production cell lines. Further, the present inventors have discovered that the growth rates /final cell densities obtainable with a given seeding density within this range may vary depending on the cell line. For example, when using HEK293 cells the inventors identified that increasing the seeding cell density from $1.10^6$ to $3.10^6$ resulted in a lower final cell density, all other conditions being equal. Thus, the method may comprise a step of determining a seeding cell density that results in the highest final cell density amongst a plurality of seeding cell densities, for a particular cell population and bioprocess.

The method may further comprise enhancing the efficiency of transformation using physical means, for example by sonoporation or electroporation. The physical means may be applied locally, i.e. to a part of the hydrogel structure.

According to a second aspect, there is provided a bioink composition comprising one or more biomaterials capable of forming a hydrogel, a cell population and a transforming agent. The bioink composition may have any of the features described in relation to the first aspect.

According to a third aspect, there is provided a cellularised structure comprising a hydrogel matrix forming a three-dimensional structure, a cell population at least partially embedded in the hydrogel matrix, and a transforming agent, wherein the cellularised structure has been obtained by depositing one or more bioink compositions each comprising one or more biomaterials capable of forming a hydrogel and a cell population and/or a transforming agent in a controlled three-dimensional shape, wherein at least one of the bioink compositions comprises a cell population and at least one of the bioink compositions comprises a transforming agent. The cellularised structure may have any of the features described in relation to the first aspect. For example, the cellularised structure may have been obtained by additive manufacturing, may comprise a first and second region, etc. According to a fourth aspect, there is provided a system for performing a bioproduction process comprising the production of a transformed cell population, the system including: a bioreactor and one or more cellularised structure(s) according to the preceding aspect. The system according to the present aspect may be configured to implement the method of any embodiment of the first aspect. The system according to the present aspect may have any of the features described in relation to the preceding aspect. In particular, the bioreactor and cellularised structure may have any of the features described in relation to the bioreactor and cellularised structure in the first aspect. The system may further comprise one of more flow devices to create a flow of medium contained in the bioreactor and in which the cellularised structure is immersed. The system may further comprise one or more support structures within the bioreactor to support the one or more cellularised structures. The system may further comprise one or more monitoring devices such as e.g. a camera, microscope, gas sensor, flow sensor, metabolite sensor, pH sensor, temperature sensor. The system may further comprise one or more supply and/or collection systems for supplying culture medium to the bioreactor and/or collecting culture medium from the bioreactor. The system may further comprise a 3D printing system for depositing a bioink composition from which the cellularised structure is obtained. The system may further comprise one or more physical transformation apparatus so as an electroporation apparatus, a sonoporation apparatus, and the like.

Brief Description of the Drawings

Embodiments of the present disclosure will now be described by way of example with reference to the accompanying drawings in which:

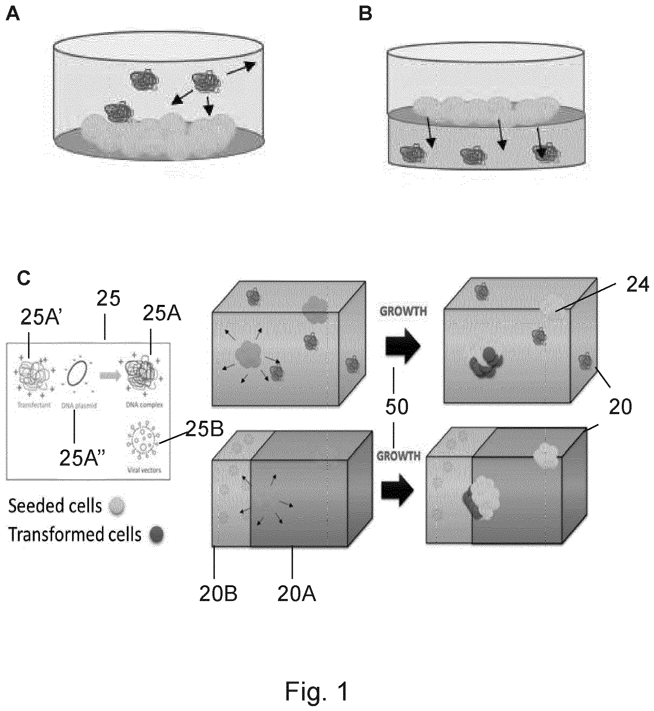

Figure 1 illustrates schematically a process for the production of cellular products (engineered cells and products thereof) according to embodiments of the invention (C) and comparative processes (A-B); (A) Transformation on a solid scaffolds or 2D cultures, by diffusion / sedimentation of DNA-transfectant complexes; (B) Reverse transformation by migration of seeded cells in a hydrogel matrix; (C) Transformation by culture of the cells embedded in a proliferative hydrogel matrix that also contains the transformation agent;

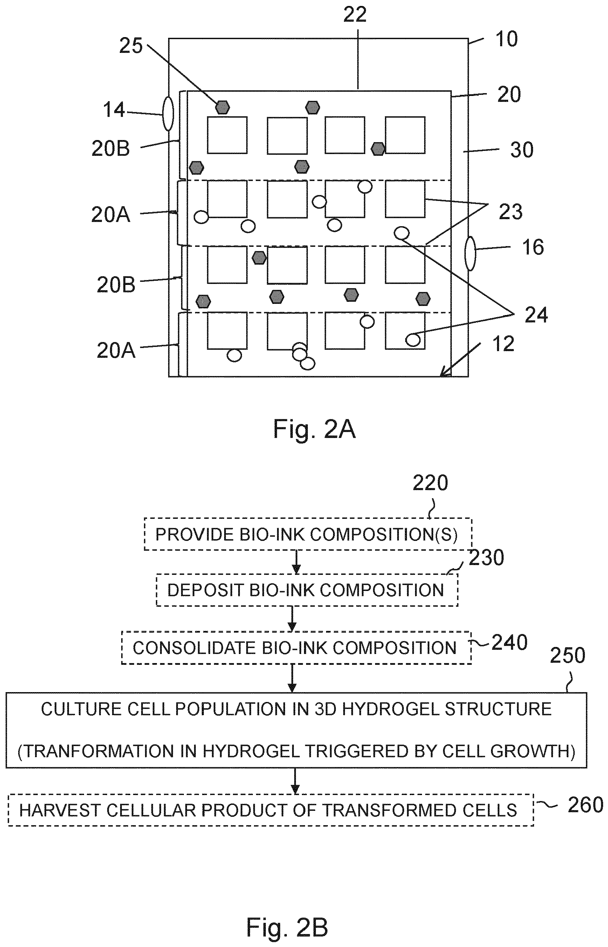

Figure 2 shows (A) components of a generic system for performing a bioprocess as described herein, and (B) a simplified process diagram for a generic bioprocess according to embodiments of the present disclosure;

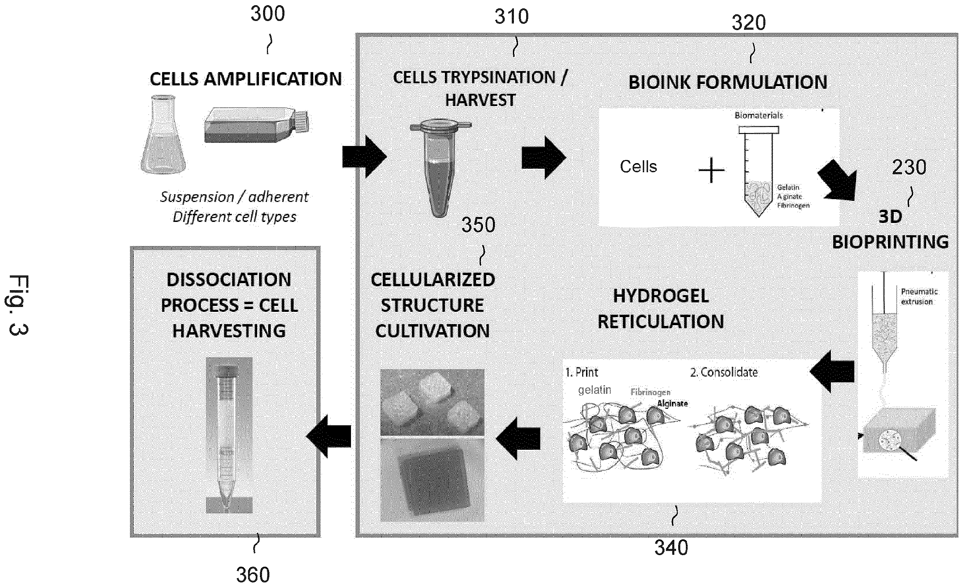

Figure 3 illustrates schematically a process for production of engineered cells and expression products thereof according to embodiments of the present disclosure;

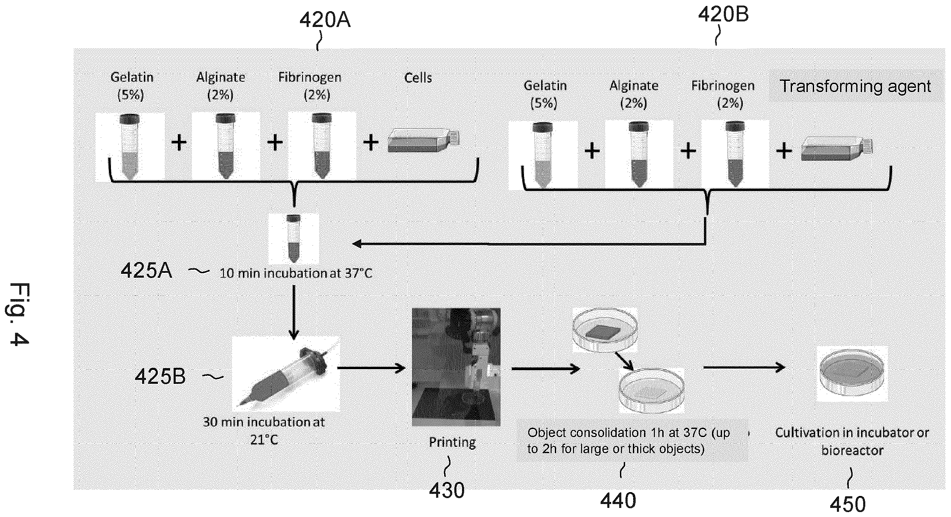

Figure 4 illustrates schematically a process for 3D bioprinting and cellularised structure consolidation according to embodiments of the disclosure;

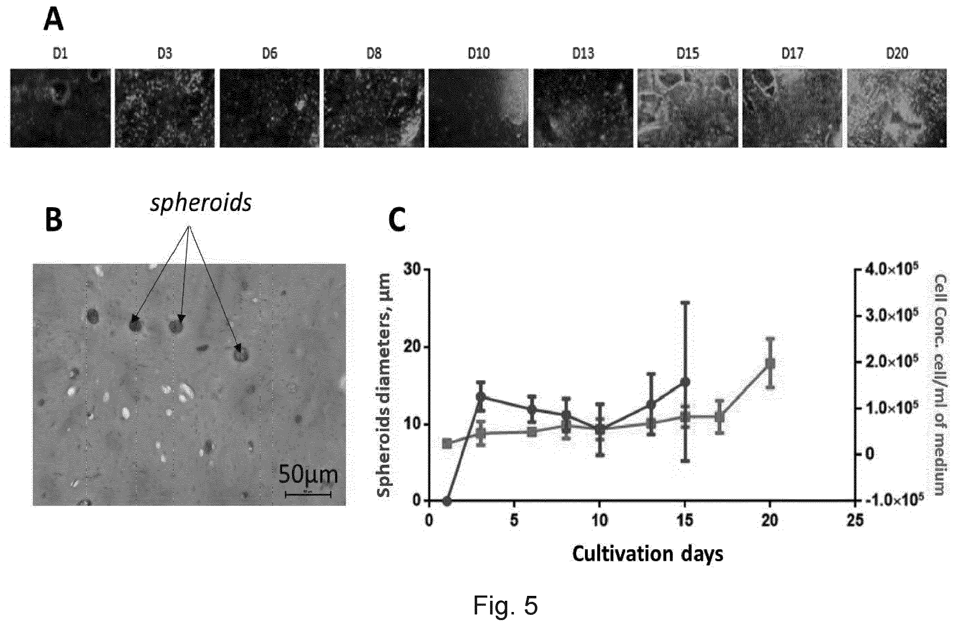

Figure 5 shows the results of an experiment in which the production of CHO cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining after 15 days of culture. C. Growth curve monitoring showing the spheroid diameters (from microscopic observation) and the cell concentration (evaluated by dissociation and counting, dissociation with collagenase 3% in PBS);

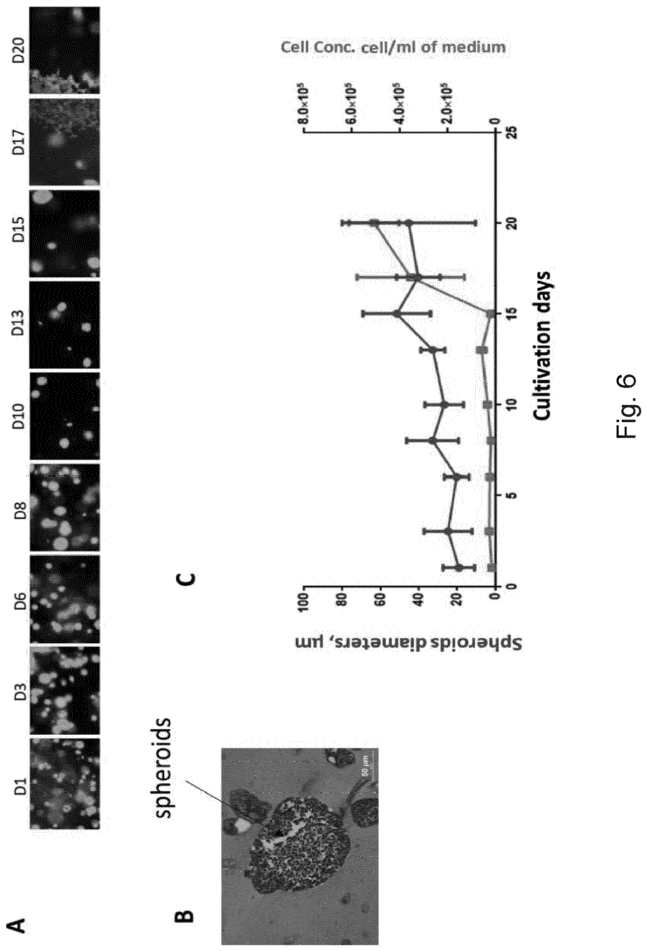

Figure 6 shows the results of an experiment in which the production of HEK cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining after 20 days of culture (cells seeded at $3 \times 10^6$ cell/mL of bioink (eq. to $3.0 \times 10^5$ cell/ mL of medium). C. Growth curve monitoring showing the spheroid diameters (from microscopic observation) and the cell concentration (evaluated by dissociation and counting, dissociation with collagenase 3% in PBS);

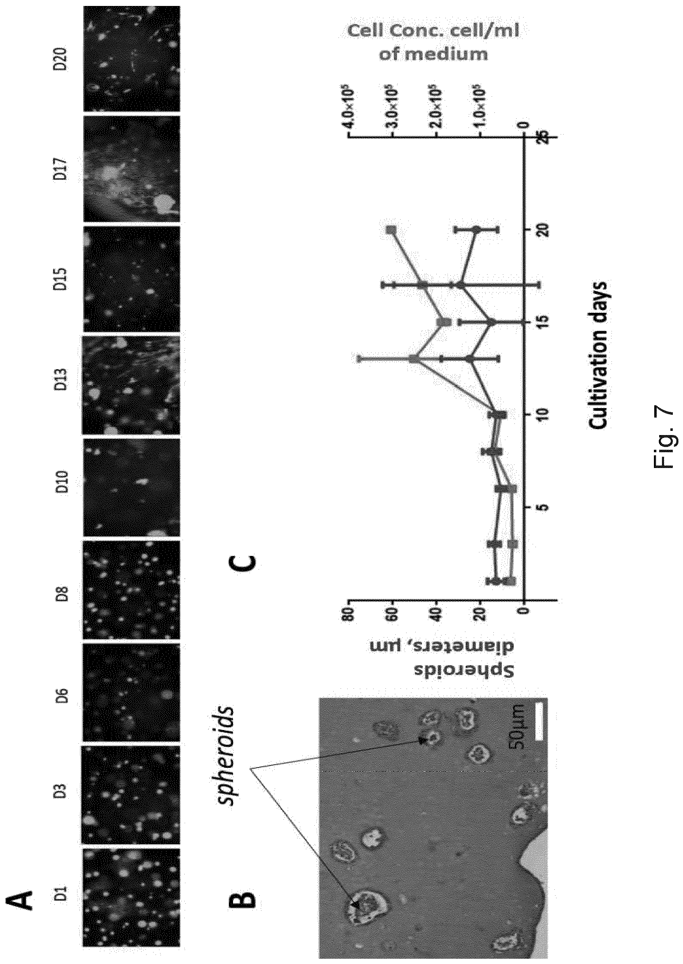

Figure 7 shows the results of an experiment in which the production of MDCK cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining after 20 days of culture (cells seeded at $3 \times 10^6$ cell/mL of bioink (eq. to $3.0 \times 105$ cell/ mL of medium). C. Growth curve monitoring showing the spheroid diameters (from microscopic observation) and the cell concentration (evaluated by dissociation and counting, dissociation with collagenase 3% in PBS);

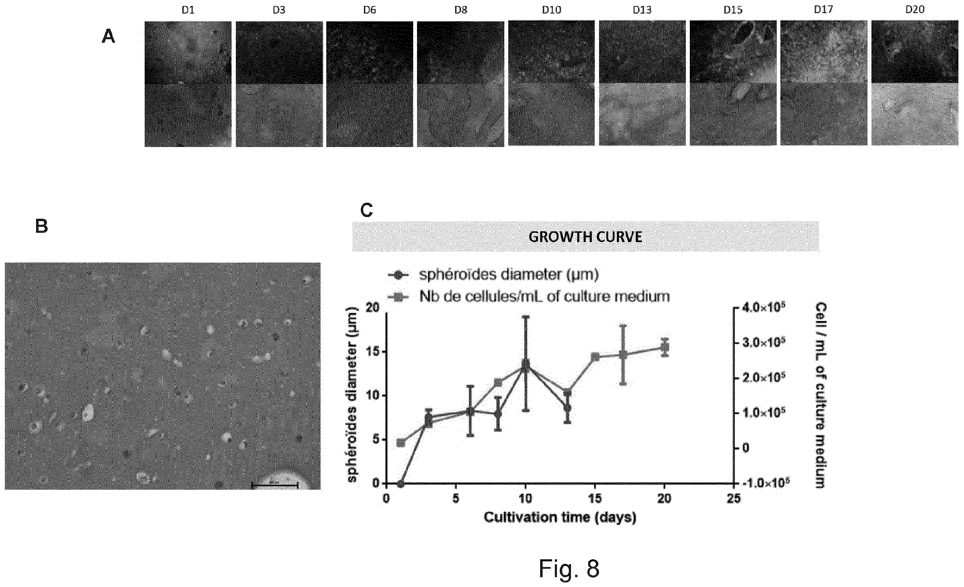

**Figure 8** shows the results of an experiment in which the production of VERO cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining. C. Growth curve monitoring showing the spheroid diameters (from microscopic observation) and the cell concentration (evaluated by dissociation and counting, dissociation with collagenase 3% in PBS);

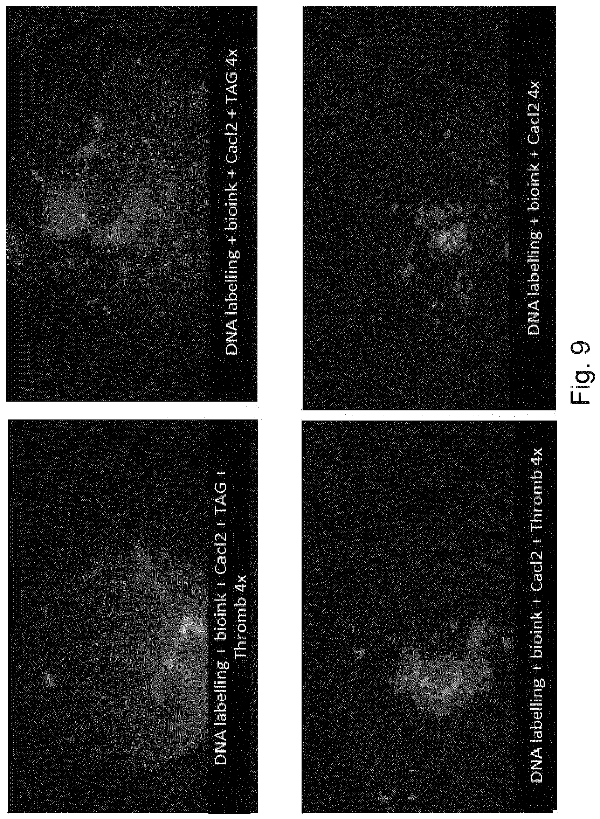

**Figure 9** shows the results of experiments demonstrating the stability of DNA within a bioink as described herein. Evaluation of the impact of consolidation solutions on the DNA stability within biomaterial over 13 days at 37°C. DNA was labelled with DAPI solution (bleu fluorescence) and observed at nm under the microscope. Droplets of biomaterial carrying labelled DNA are presented in the following images in different consolidation conditions (CaCl$_2$ 3%; TAG : Transglutaminase 4%; Throm : Thrombin 10U/ml);

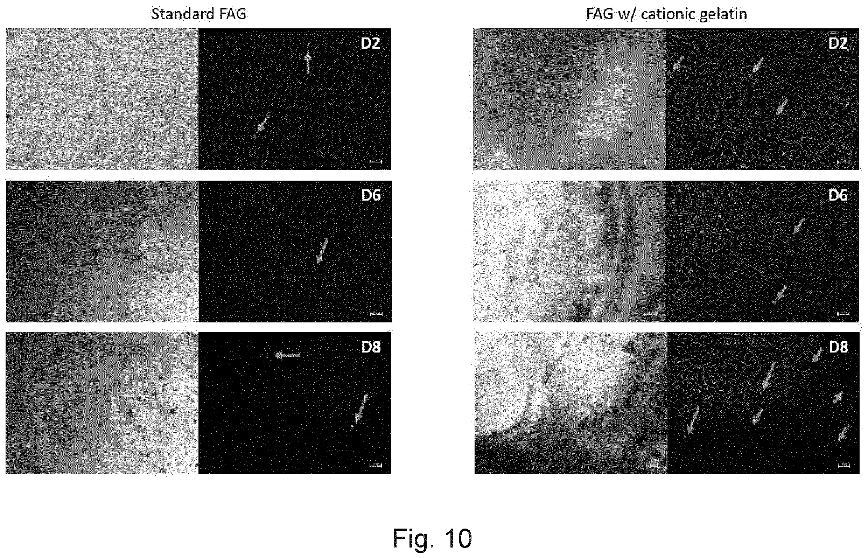

**Figure 10** shows the results of experiments demonstrating long-lasting transformation by transfection according to the present disclosure. HEK293 cells cultured in 3D molded biomaterial at cell density of $3 \times 10^6$ cell/ml with 17μg of GFP plasmid (pEGFP-C1) per milliliter of biomaterial. The microscopic observations were performed along the cultivation period after 2-, 6- and 8-days post-molding;

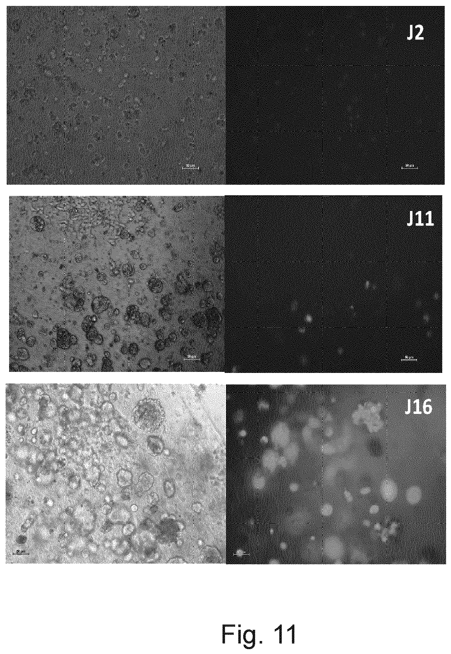

**Figure 11** shows the results of experiments demonstrating the transformation by transduction of mammalian cells with GFP-lentivirus embedded in a proliferative biomaterial during cell growth, as described herein. Cultivation is lasting for over 15 days. HEK293 cells 3D printed at cell density of $3 \times 10^6$ cell/ml with GFP-lentivirus.

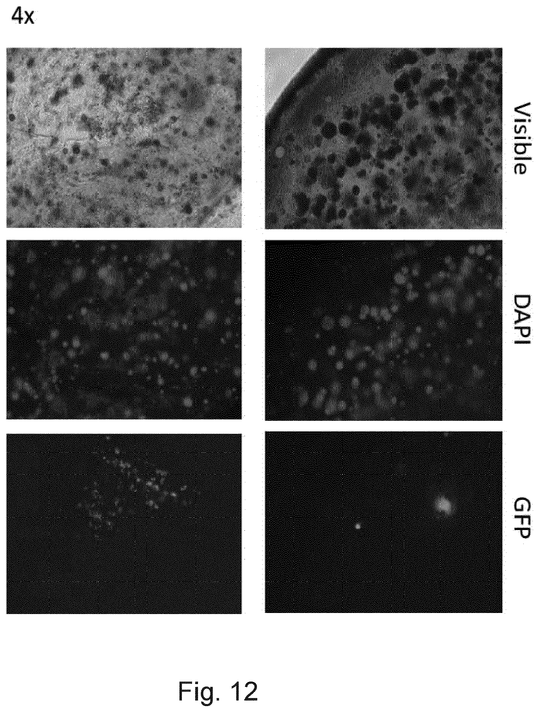

**Figure 12** shows the results of experiments demonstrating the transformation by transfection of mammalian cells with a GFP encoding plasmid. HEK293 cells were grown embedded in a bioink 3D scaffold also comprising a DNA plasmid encoding for the GFP fluorescent protein. Observation after 12 days of cultivation;

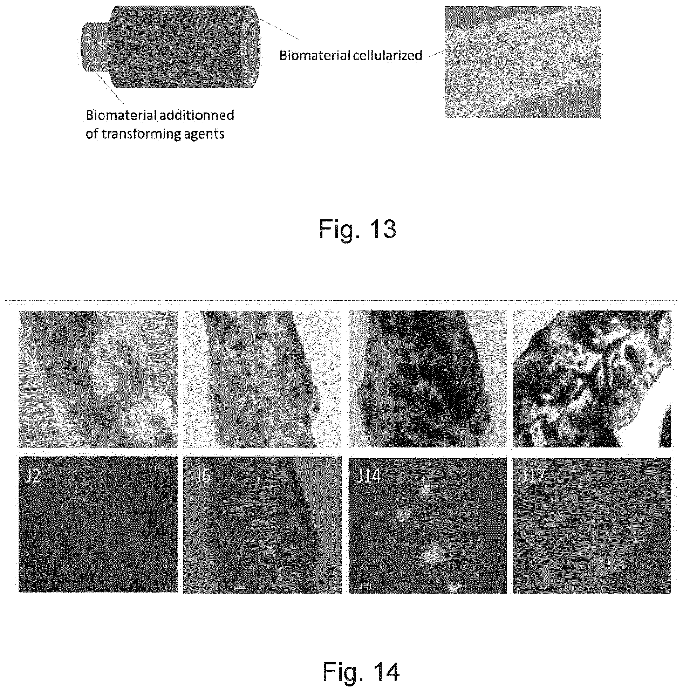

**Figure 13** illustrates schematically a process for the production of engineered cells and expression products thereof according to embodiments of the invention where the cells are provided embedded in a biomaterial surrounded by a biomaterial comprising the transformation agent;

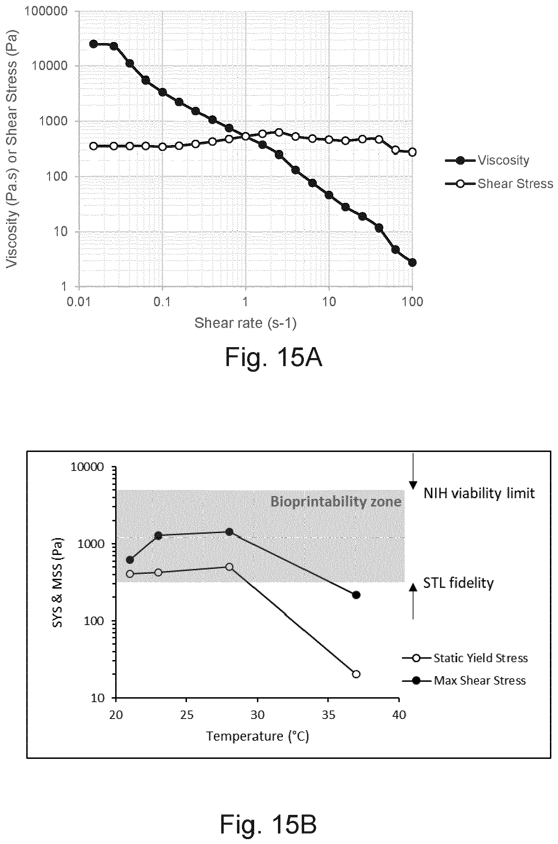

**Figure 14** shows the results of experiments demonstrating the use of localised transforming agents as illustrated on Figure 15 resulting in long-lasting transduction. Transformation of HEK293 cells with GFP-lentivirus over 17 days of cultivation. Cells and viral vectors were separated in two distinct compartments (internal = viral vectors; external = cells). This experiment demonstrates only growing cells are transformed over time when they are in contact with the transforming agent embedded and stabilized within the biomaterial. Consolidation was performed with transglutaminase, thrombin and CaCl$_2$;

**Figure 15** shows exemplary results for a process of determining the rheological properties of a bioink composition and printing conditions associated with rheological properties suitable for printing. A. Example reference rheogram for a bioink composition according to the disclosure, with rheological properties suitable for printing (maintain shape fidelity and cell viability). B. Representation of the bioprintability zone for a bioink formulation with rheological properties as in A. Here different rheological behavior was tested with regards to the shape fidelity of the bioprinted object (STL fidelity) and with regards to the viability of cells after the microextrusion process (NIH fibroblast cell viability). The shaded zone represents the shear stress (MSS) and static yield stress (SYS) that cells can handle.

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures.

As used herein, the term "bioprocess" (also referred to herein as "biomanufacturing process") refers to a process where biological components such as cells, parts thereof such as organelles or multicellular structures such as organoids or spheroids are maintained in a liquid medium (typically either in suspension or supported on a scaffold) in an artificial environment such as a bioreactor, so that they can perform a function (such as e.g. the production of a biomaterial). According to the present invention, a cell population may be maintained at least partially embedded in a hydrogel matrix forming a three-dimensional structure (referred to herein as "cellularised object" or "deposited object"). The cell population may be at least partially embedded in the hydrogel matrix in that at least a portion of the cell population grows within the hydrogel matrix as opposed to on the surface thereof. Typically, the cells growing within the hydrogel matrix grow as spheroids. Part of the cell population may also grow on the surface of the hydrogel matrix. This may be removed if desired, for example, if the cells that grow on the surface of the cellularised object interfere with the control of the flow through the structure. A bioreactor is a device in which a biological component can be maintained in controlled environmental and operating conditions (e.g. pH, temperature, pressure nutrient supply, and waste removal). A bioreactor typically comprises at least a vessel suitable for holding a liquid culture medium. Thus, as used herein, the term "bioreactor" does not necessarily refer to a standard stirred tank for suspension cultures, also such vessels are encompassed by the term. The configuration, shape and/or volume of the bioreactor may depend in the bioprocess. The vessel itself may be 3D printed. The vessel may be a single use vessel, or a reusable vessel. The vessel may be made primarily of stainless steel, plastic or any other material known in the art for use in industrial bioprocesses. The volume of the vessel may be determined based on the requirement of the bioprocess, such as e.g. the desired number of cells at the end of the bioprocess. As another example, the volume of the vessel may be determined based on the size of the cellularised object(s) and/or the configuration of the supports (in embodiments comprising one or more cellularised objects cultured on a support in a vessel), for example to ensure that the cellularised object(s) is/are fully immersed in culture medium and/or to obtain a ratio of volume of culture medium to volume of cellularised object within a desired range and/or to obtain number of cells in the cellularised object per volume of culture medium within a desired range. For example, it may be advantageous for the ratio of volume of culture medium to volume of the cellularised object(s) / volume of the bioink used to obtain the cellularised object(s) to be between 5 and 50, preferably below 50, such as e.g. 10, 20, 30 or 40 (e.g. 10 ml of culture medium for every $cm^3$ of cellularised object / deposited bioink). As another example, it may be advantageous for the number of cells per ml of culture medium in the vessel to be at least $10^4$ cell/ml (such as e.g. between 1.0E+04 cell/ml and 1.0E+08 cell/ml, preferably at least 1.0E+05 cell/ml) at the start of the culture (i.e. seeding density). It may further be advantageous for the number of cells per ml of bioink used to make the cellularised object(s) to be at least $10^6$ cells/ml. The present inventors have identified that such cell densities lead to better growth rates and ultimately higher final cell numbers. Within the context of the present invention, the bioprocess comprises a cell culture. A bioprocess typically results in a product, which can include biomass and/or one or more compounds that are produced as a result of the activity of the biological components. A bioreactor can be a single use vessel or a reusable vessel in which a liquid medium suitable for carrying out a bioprocess can be contained. The present invention is applicable to any type of bioreactor and in particular to any vendor and any scale of bioreactor from benchtop systems to manufacturing scale systems.

A cell culture refers to a bioprocess whereby live cells are maintained in an artificial environment such as a bioreactor. The methods and systems described herein are applicable to bioprocesses that use any types of cells that can be maintained in culture, whether eukaryotic or prokaryotic. The cells may be primary cells, cell lines or production cells (such as cells from a cell line that is a production cell line). A cell line is a population of immortal cells maintained in artificial (culture) conditions. In embodiments, the cells are "production cells". Production cells are cells from cell populations developed for the production of biomaterials and that typically have higher growth rates and/or protein expression rates and/or metabolic rates than primary cells of the same origin. Production cells are typically from production cell lines. Production cells may be derived from primary cells, such as e.g. mesenchymal stem cells derived from bone marrow, which may be used in particular to produce exosomes and extracellular vesicles. Such cells may have good capacity for in vitro expansion and may be used for the production of cells of particular cell types and/or for production of proteins of interest. The cells may be animal cells, such as mammalian or insect cells. In embodiments, the cells may be animal cells that are not insect cells. In embodiments, the cells may be mammalian cells. In embodiments, the cells are all from the same cell type, preferably the same cell line. The cells may be selected from MDCK cells (Madin-Darby Canine Kidney cells, which are epithelial cells from dog kidney), VERO cells (epithelial kidney cells from green monkey), AGE.CR1™ (ProbioGen, Muscovy duck retina cells designed for virus-based vaccine production), PRE.C6 (a cell line derived from human embryonic retinal cells), EB.14 (a chicken embryonic stem cell line), EP.66™ (a duck embryo-derived cell line), HEK293 (a human embryonic kidney cell line), BHK21 (a baby hamster kidney cell line), CHO (Chinese hamster ovary cell lines), NS0 (a murine myeloma cell line), Sp2 (a cell line derived from the fusion of a BALB/c mouse spleen cell and a mouse myeloma cell line), Sf9 (a clonal isolate of *Spodoptera frugiperda* Sf21 fall armyworm moth cells), SF21 (a cell line derived from ovarian *Spodoptera frugiperda* fall armyworm moth cells), MRC-5 (human normal lungs fibroblasts), WI-38 (human normal lungs fibroblasts), CEF (chicken embryo fibroblasts), hybridomas (murine antibody producing B-cells fused with myeloma cells). In embodiments, the cells are selected from VERO cells, CHO cells, MDCK cells, and HEK293 cells. Successful growth of each of these cell lines in the context described herein has been demonstrated by the inventors.

A product of a bioprocess (also referred to herein as "cellular product", "biomaterial" or "target biologic") may include a metabolite, a cell or a part thereof (such as e.g. an organelle), a population of cells (e.g. a tissue), a virus or viral particle, a desired protein (e.g. an antibody, an immunoglobulin, any recombinant protein), a toxin, one or more by-products, or any other type of molecule manufactured using a bioprocess. There may be more than one cellular products of interest. For example, the cells may secrete a protein of interest that may be harvested from the culture medium, and the cells themselves may also represent a product of interest that may be harvested at the end of the culture. As another example, the cells may produce a plurality of proteins and/or metabolites of interests, some of which may be secreted in the culture medium (and e.g. harvested during the culture), and some of which may be retained in the cells (and harvested at the end of the culture, e.g. through cell lysis and product purification). The cellular product may be a biopharmaceutical, also referred to as "biologics", for example an exogeneous protein expressed by engineered cells. The cellular product may be a virus, such as e.g. an attenuated or non-replicating viral particle for use as a viral vaccine. The present invention may be particularly advantageous in the context of the production of a biologic or virus because it may ensure long lasting efficient transfection alongside cell growth, thereby resulting in non-transient production of the cellular product of interest, over relatively long production time scales (e.g. 15 days or more) without having to repeat a transformation protocol. The cellular product may be a tissue. For example, a bioprinted tissue may be used in the context of regenerative medicine. The present invention may be particularly advantageous in the context of tissue engineering (for example for regenerative medicine), as it enables the localisation of transformation as well as its timing to be precisely adapted to ensure a particular pattern of transformation that is conducive to the formation of a particular tissue of interest. For example, the localised modification of cells within a growing printed tissue may enable defined cells to secrete growth factors, chemokines and/or differentiation factors to produce complex tissues *in vitro*. Further, the localised modification of cells within a printed tissue may also enable to obtain heterogeneous tissues comprising cells that model healthy tissues and cells that model diseases tissues in specific, predetermined patterns. This may be particularly useful in contexts such as drug testing.

The invention relates at least in part to the use of a 3D hydrogel structure in which cells are embedded, and in the culture of said cells in such a proliferative hydrogel matrix. The 3D hydrogel structure is typically obtained through a two-step process that involves forming a composition comprising biomaterials capable of forming a hydrogel into a 3D shape (also referred to as "deposited object" or "cellularised structure/object") and consolidating the object so that it maintains its shape in culture. The step of forming the composition can be performed by molding (casting, etc.), or by 3D printing. Thus, the terms "deposited object" and "depositing" do not necessarily refer to an object that has been 3D printed, but to any object that has been formed into a 3D shape. The step of forming the composition is preferably performed by 3D printing. Similarly, the term "deposited object" does not necessarily refer to an object that is deposited onto a surface, wherein the object is also supported on a surface throughout its use, e.g. in culture. For example, a deposited object can be formed on a surface and subsequently cultured in suspension. The term "3D printing" (also referred to as "additive manufacturing") refers to the creation of a 3D structure by computer controlled deposition of a material. Within the context of the present invention, the material is one or more compositions comprising biomaterials capable of forming a hydrogel and either cells, a transforming agent or both, also referred to as a bioink, as described below. For simplicity, the term cellularised structure/object will be used to refer to deposited objects that are formed from one or more compositions all comprising cells as well as for deposited objects that are formed from one or more compositions not all of which comprise cells, although not all parts of the latter type of objects may in fact comprise cells. Thus, a celularised object/structure comprises cells embedded in a 3D hydrogel matrix, but not all regions of such an object/structure may comprise cells.

3D printing involves the controlled deposition of material into a three-dimensional shape. 3D printing typically follows a computer-aided design (CAD) model. CAD models can be stored as STL (stereolithography file format) files or AMF (Additive Manufacturing File format) files. 3D printing may for example be achieved by extrusion (for example, using a syringe with a piston or screw) or by ink-jet (by projection of ink droplets). Within the context of the present disclosure, 3D printing is typically achieved by extrusion, particularly pneumatic extrusion. However, 3D printing by inkjet is also possible and explicitly envisaged. For example, a bioink may be loaded into an extrusion syringe that is mounted on a bioprinter. The syringe may be equipped with a pusher to control the flow of bioink. As described further below, the bioink may be in a flowable consistency during printing, and may be rapidly solidified when deposited by the 3D printer. This ensures that the printed structure (also referred to as "deposited object" or "cellularised structure/object") maintains its 3D shape. A similar process may be used during molding, where the bioink may be prepared in a flowable consistency and poured or otherwise transferred to a mold, where is it rapidly solidified. Once solidified, the bioink may be further consolidated by polymerisation, as will be explained further below. In the context of 3D printing, the deposited object is typically formed by progressive addition of material in the form of beads of material or a filament of material. The deposited object may be formed from filaments of material that are between 200 and 800 microns in diameter. The present inventors have found such dimensions to be particularly suitable for the culture of cells at high density. Without wishing to be bound by theory, the inventors believe that this may be due to higher amounts of cell growth occurring on the surface of filaments than within filaments, such that a balance can be obtained between stability (and protective nature) of the structure and growth of the cells within the above-mentioned range of dimensions.

When using 3D printing to form the deposited object, the deposited object may have an internal three-dimensional structure. In other words, the deposited object may not be a solid form such as a solid cube. This may also be referred to as the deposited object comprising cavities or pores. Thus, the deposited object may be referred to as having a certain porosity.

The porosity of a deposited object may refer to the ratio of the volume of the pores to the total external volume of the deposited object. When using 3D printing to form a cellularised object, the porosity of the cellularised object can advantageously be tightly controlled through control of the deposited shape. Further, this can be easily varied by changing the shape that is being printed. In embodiments, the pore sizes can be between 0.1 and 2 $mm^2$, where the surface refers to the area of the cross section of a channel-shaped pore. In embodiments, the pores can have a cross section size between 500 and 2000 $\mu m$, where the diameter of a pore refers to the diameter of the smallest circle that fits the entire cross-section of the pore. The pores may be in the form of channels with a constant cross section. For example, a square cross section may be used. Pores with a square cross section may for example have a cross section with sides between 400 and 1200 $\mu m$ (leading to a diameter between approximately 566 and 1697 $\mu m$). The total volume of the deposited object refers to the external volume of the deposited object. Advantageously, when using 3D printing the total volume of the deposited object may be varied, as well as the external shape of the object and its internal structure, to suit a particular application (such as e.g. a particular scale of production, a particular cell type, a particular bioreactor, etc.). This advantageously may not require changing the material used and/or the hardware used for printing. A variety of shapes and dimensions for the deposited object are envisaged. In embodiments, the deposited object has dimensions such that the total volume of the deposited object is in the order of $cm^3$ (e.g. 1 to 10 $cm^3$) or $dm^3$ (e.g. 1 to 10 $dm^3$). For example, the three-dimensional structure may have been obtained by depositing between 1 ml and 1l of bioink, between 1 ml and 500 ml of bioink or between 1 ml and 250 ml of bioink. Deposited objects with dimensions in these ranges may advantageously enable to obtain cell densities (in number of cells per ml of culture medium in the vessel) similar or higher to those achievable in cell production processes in perfused cell culture. For example, standard perfusion processes are commonly operated at 20-35 $\times 10^6$ cells/ml in 4l cultivation vessels. According to the present invention, cells are cultured at least partially embedded within a three-dimensional structure comprising a hydrogel matrix, which provides a protection to the cells and enables to maintain the cells inside of the vessel without the use of a separation process. This enables to reach even higher cell densities than is possible in a standard perfusion process where the cells are cultured in suspension. For example, cell densities

of $10^7$ cells/ml (volume of bioink), $10^8$ cells/ml (volume of bioink) or more may be reached (such as e.g. up to $10^9$ cell/ml or $10^{10}$ cell/ml of bioink), depending at least in part on the porosity of the object and the size of the cells cultured. For example, assuming that cells have an average diameter between 7.15 to 9.14 $\mu m$ (as measured for CHO cells by Sakhr et al., Int J Mol Sci. 2021 Apr; 22(7): 3290, 2021), and assuming a porous deposited object with 50% porosity, the maximal cell density that could be reached may be of the order of 7.5 $\times 10^9$ cell/$cm^3$ (or 7.5 $\times 10^9$ cell/ml) of bioink. Thus, the same number of cells as typically cultured in a 4l cultivation vessel (35 $\times 10^6$ cell/ml) can be obtained using a deposited object that was made from 250 ml of bioink. As explained above, such a deposited object may be cultured in a vessel comprising a ratio of culture medium to volume of deposited object (volume of bioink) between 5 and 50, i.e. in this case a vessel of between 1.25 l and 12.5 l. Such three dimensional structures may be supported on a surface. For example, the deposited object may be a cube or parallelepiped. In embodiments, the deposited object may have a three-dimensional lattice structure. Preferably, the lattice structure is a regular lattice structure. For example, the deposited object may have a regular three-dimensional cubic lattice structure. The regular cube lattice structure may comprise cubic pores separated by walls, or parallel channels of square section extending through one dimension of the object. This advantageously results in a simple structure that is relatively easy to print, enables a homogeneous flow of liquid through the structure and comprises pores of homogeneous sizes, thereby increasing the likelihood that the cell culture within the structure will be homogeneous. In embodiments, the deposited object has a plurality of porous external walls (also referred to as "sides" or "faces") and a plurality of solid external walls. This may enable a controlled flow of liquid (such as e.g. culture medium) through the deposited object, as a flow can only enter and exit the deposited object through the porous external walls. For example, the deposited object may have two substantially parallel porous external walls, enabling a parallel flow through each of the two porous external walls. The other walls may be solid in order to guide the flow through a first porous external wall, through the object and through a second porous external wall. Alternatively, the three-dimensional structure may have dimensions such that the total (external) volume of the three-dimensional structure is in the order of $\mu m^3$ (e.g. structures with an external diameter of between 50 $\mu m$ and 500 $\mu m$, or with an external volume of $65.10^3$ to $65.10^6$ $\mu m^3$ or 0.065 $mm^3$) to $mm^3$ (e.g. 1 to 1000 $mm^3$). Such three dimensional structures may be cultured in suspension. Such three dimensional structures may be deposited as droplets rather than filaments.

The deposited object may be incubated in a vessel also called a "bioreactor". The bioreactor may be equipped with one or more fluid inlet(s) and one or more fluid outlet(s). The fluid inlets and outlets may enable the provision of nutrients, gasses etc. to the cells in the deposited structure, by creating a flow of medium through the deposited object. The fluid inlet(s) and outlet(s) may respectively be connected to a supply tank and collection system. The flow at the inlet(s) and/or outlet(s) may be constant or variable during the bioprocess, whether in composition or in volumetric flow. For example, the flow of liquid through the bioreactor (assuming that the volume of liquid flowing into the bioreactor is equal to the flow volume of liquid flowing out of the bioreactor) may be increased one or more times during the bioprocess, to support the growing number of cells. The deposited object may be positioned relative to a flow inlet and outlet of the bioreactor in which the deposited object is incubated so as to optimise the characteristics (e.g. direction, speed etc.) of the flow of liquid through the deposited object. The flow of liquid at the inlet(s) and outlet(s) may be controlled for example to maintain the fluid velocity at the inlet/outlet within a predetermined range. This range may be adapted for each inlet/outlet in order to optimise the flow of liquid through the deposited structure, for example to ensure adequate supply of nutrients / gasses while maintaining cell viability by controlling mechanical stress to the cells within the deposited structure.

As used herein, the terms "computer system" of "computer device" includes the hardware, software and data storage devices for embodying a system or carrying out a computer implemented method, such as e.g. for printing a 3D object as part of a 3D printing system. For example, a computer system may comprise one or more processing units such as a central processing unit (CPU) and/or a graphical processing unit (GPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. For example, a computer system may be implemented as a cloud computer. The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The term "hydrogel" refers to a crosslinked hydrophilic polymer that does not dissolve in water. Within the context of the present invention, a hydrogel may be obtained by consolidation (also referred to as "polymerisation" or "cross linking") of a bioink. A bioink is a composition comprising one or more biomaterials that can form a hydrogel in appropriate polymerisation condition, and one or more cells or one or more transformation agents. Suitably, a bioink as described in US 2019/002836A1 may be used, the entire content of which is incorporated herein by reference. A biomaterial is a biocompatible material, i.e. a material that is compatible with the maintenance of living cells. In embodiments, the one or more biomaterials that can form a hydrogel comprise alginic acid or a sodium salt thereof also referred to as "sodium alginate" or "alginate" (a naturally occurring polysaccharide which can form a polymer called "alginate" in the presence of e.g. Calcium ions, a process also called "gelation"), fibrinogen (a naturally occurring glycoprotein complex which can form a polymer called "fibrin" by enzymatic conversion by thrombin, a process also referred to as "coagulation"), and/or gelatin (a peptide composition that can form a hydrogel when hydrated and maintained at temperatures below gelling point). In embodiments, the one or more biomaterials that can form a hydrogel comprise chitosan. For example, the one or more biomaterials that can form a hydrogel may comprise chitosan, alginate and fibrinogen. As another example, chitosan may be the only biomaterial capable of forming a hydrogel that is used in the bioink. At least one of the biomaterials is such that the polymerised object is stable in the desired cell culture conditions. For example, when the cell culture conditions comprise a temperature T, at least one of the biomaterials is such that it remained polymerised at temperature T. As another example, when the cell culture conditions comprise a concentration of one or more components (such as e.g. ions) within a certain range, at least one of the biomaterials is such that it remains polymerised in media having said composition. As another example, when the cell culture conditions require a pH within a certain range, at least one of the biomaterials is such that it remains polymerised in media having a pH within said range. It is advantageous for at least one of the biomaterials used to be such that polymerisation is not reversible, in order to ensure the stability of the hydrogel in culture conditions. For example, the coagulation of fibrinogen is irreversible, whereas the gelling of alginic acid can be at least partially reversed through removal of calcium ions, and the gel formed by gelatin can be melted by reheating. Other biocompatible materials that can form a hydrogel may be used. The one or more biomaterials may comprise one or more biomaterials that can form a hydrogel, and one or more additional components or additives. The one or more additional components may provide one or more desired properties to the bioink and/or the hydrogel. In embodiments, at least one of the biomaterials and/or one of the one or more additional components ensures that the bioink has desired rheological properties (such as e.g. a desired consistency and/or viscosity) prior to and during deposition. For example, the composition may comprise one or more components that ensure that the bioink can maintain its shape at least temporarily, e.g. during printing, prior to the irreversible consolidation of the biomaterials into a hydrogel. The rheological properties of the bioink and the associated conditions for printing that enable the bioink to maintain its shape at least temporarily while being compatible with cell viability may be assessed for example as described in the examples. The one or more additional components may also be biomaterials. For example, the bioink may comprise a gelling agent, such as e.g. gelatin. A gelling agent such as e.g. gelatin may be used to maintain the shape of the bioink for example during deposition, by cooling the deposited bioink. The bioink may then be treated with a polymerisation solution, also referred to as "consolidation solution" (or a plurality of solutions) in order to cause the other biomaterials to form a hydrogel. When the bioink comprises alginic acid, the polymerisation solution(s) may comprise calcium ions. When the bioink comprises fibrinogen, the polymerisation solution(s) may comprise thrombin. In embodiments, the polymerisation solution(s) may comprise transglutaminase (TAG). The present inventors have identified that it is advantageous for the consolidation solution to comprise transglutaminase as this may enhance the long term stability of transforming agent (in particular DNA, such as e.g. plasmid DNA) in the deposited object. It is advantageous for the gelling agent to have a gel point (also referred to as "transition point" or "gel melting temperature") that is compatible with cell viability, i.e. that is such that there exists a range of temperatures compatible with cell viability that is below the gel point of the gelling agent, and a range of temperatures compatible with cell viability that is above the gel point of the gelling agent. For example, the gelling agent may have a melting temperature below 35°C. For example, a gelatin with a gel point between 27 and 32 °C (such as e.g. between 28 and 30°C) may be used. Thus, the bioink may be deposited while composition is a temperature that is above the gelling point of the composition (such as e.g. between 28 and 37°C) or slightly below the gelling point of the composition (such as e.g. between 21 and 28°C), such that the bioink is fluid, and rapidly cooled at / after deposition to a temperature that is below the gelling point of the composition after deposition (such as e.g. between 0 and 20°C) such that the bioink gels. This may be achieved for example by depositing the bioink onto a cooled substrate. The temperature of the cooled substrate and/or the environment in which the bioink is deposited may be adjusted depending on the temperature of the bioink and the rate of deposition, in order to ensure that the deposited object maintains its shape. In embodiments, the bioink may be deposited while the composition is a temperature between 21 and 28°C. In embodiments, the consolidation of the other biomaterials is performed at a temperate above the gelling point of the gelling agent. This may re-dissolve at least part of the gelling agent which can then be removed, when the other biomaterials have been polymerised. This may further be advantageous in optimising the activity of any enzyme that is active during consolidation. For example, a temperature of approximately 37°C is advantageous for consolidation with thrombin. The bioink may be obtained by mixing a plurality of solutions, each comprising a biomaterial capable of forming a hydrogel and/or a transformation agent and/or a cell population. For example, the bioink may comprise a first solution comprising gelatin, a second solution comprising alginate, and a third solution comprising fibrinogen. One or more of these solutions may further comprise a transformation agent. Advantageously, the solution comprising the transformation agent is cationic. For example, the transforming agent may be included in the first solution comprising gelatin. As another example, the transforming agent may be included in the second solution comprising alginate. When one or more of the biomaterials are modified biomaterials (such as e.g. cationised gelatin or cationised alginate), the transforming agent may be included in the solution comprising the modified biomaterial. The bioink may comprise a first solution comprising between 5% and 40% w/v (weight by volume) of gelatin (such as e.g. approximately 20% w/v of gelatin), a second solution comprising between 1% and 12% w/v of alginate (such as e.g. approximately 4% w/v of alginate), and a third solution comprising between 1% and 15% w/v of fibrinogen (such as e.g. approximately 8% by mass fibrinogen). The first solution may represent 35 to 65% by volume of the bioink (such as e.g. approximately 50% by volume). The second solution may represent 15 to 35% (such as e.g. approximately 25%) by volume of the bioink. The third solution may represent 15 to 35% (such as e.g. approximately 25%) by volume of the bioink. Thus, the bioink may comprise between 1.75% and 26% w/v of gelatin, between 0.15% and 4.2% w/v of alginate (e.g. sodium alginate), and between 0.15% and 5.25% w/v of fibrinogen. For example, the bioink may comprise approximately 5% w/v gelatin, 2% w/v alginate and 2% w/v fibrinogen. The bioink and each of the solutions may comprise the (respective) biomaterials in a solution comprising nutrients and one or more components ensuring an osmotic pressure compatible with cell viability. In other words, the bioink has an osmotic pressure compatible with cell viability. Thus, the bioink may comprise a solution that preserves the osmotic pressure of the cells in the bioink. For example, the bioink may have a NaCl content between 0.2% and 5% w/v. As another example, the bioink may be obtained by mixing solutions prepared using a growth medium. Preferably, the solution that preserves the osmotic pressure of the cells in the bioink is a nutritive solution. For example, the bioink may comprise a culture medium solution. A culture medium (also referred to as "growth medium") solution is a solution designed to support the growth of a population of cells. Such a solution is typically sterile, and comprises substances required for the growth of cells. Examples of culture media are known in the art and include, DMEM, RPMI 1640, MEM, F-12 K, etc. The growth medium may be a calcium free growth medium. For example it may be calcium free DMEM, herein referred to as DMEM(-). In embodiments, the bioink composition comprises one or more solutions (as explained above), each of which comprises one or more biomaterials and/or cells, in a nutritive solution that preserves the osmotic pressure of the cells (such as e.g. a culture medium, e.g. DMEM(-)). For example, the first solution mentioned above may comprise between 5% and 40% w/v (weight by volume) of gelatin in culture medium (e.g. DMEM(-)). Similarly, the second solution may comprise between 1% and 12% w/v of alginate in culture medium. The third solution may comprise between 1% and 15% w/v of fibrinogen in culture medium. The precise culture medium used may depend on the cells to be cultured. The growth medium may be a calcium free growth medium. For example, the first solution mentioned above may comprise between 5% and 40% w/v (weight by volume) of gelatin in culture medium (e.g. DMEM(-)). Similarly, the second solution may comprise between 1% and 12% w/v of alginate in culture medium. The third solution may comprise between 1% and 15% w/v of fibrinogen in culture medium. The precise culture medium used may depend on the cells to be cultured. The bioink may be consolidated using a polymerisation solution. When the bioink comprises alginate and fibrinogen, the polymerisation solution may comprise calcium ions and thrombin. The polymerisation solution may comprise between 1% and 5% w/v of a calcium salt (such as e.g. 3% w/v $CaCl_2$) and between 2 and 40 U/mL (such as e.g. approximately 10 U/mL) of thrombin. Other biomaterials that can be used instead or in addition to these include e.g. chitosan and derivatives thereof, as well as derivatives of the above-mentioned biomaterials. For consolidation, the deposited bioink may be incubated in the polymerisation solution for a predetermined period of time (such as e.g. 1 hour), for example by immersion of the deposited object in the polymerisation solution. Immersion of the deposited object in the polymerisation solution may advantageously result in a homogeneous polymerisation of the biomaterials. The bioink composition may be prepared for use from one or more stable aqueous solutions (such as e.g. the first, second and third solutions described above), and a suspension or pellets of cells. When a suspension of cells is used, the suspension may be in a culture medium. When alginate is used as one of the biomaterials, the suspension of cells (and every other solution used to prepare the bioink) is preferably calcium free. For example, a calcium free culture medium such as DMEM(-) may be used to suspend the cells prior to mixing with the other components of the bioink. When the deposited object has been polymerised, the cells within the object may then be cultured in this environment. The cells may therefore be contained within and protected by a three-dimensional network formed by the consolidated biomaterials themselves, and any three-dimensional structure formed by the shape of the deposited object.

According to the present invention, the hydrogel formulation (or the bioink composition from which the hydrogel is obtained) may comprise one or more components that enhance the efficiency of transformation and/or the stability of a transforming agent. The one or more components may be in the form of one or more additives and/or in the form of a biomaterial that is also capable of forming a hydrogel (in which case the hydrogel or bioink may be referred to as a "modified matrix"). An additive as used herein refers to a compound, complex or component that can be added to a hydrogel or bioink formulation and that does not form a hydrogel. Additives that may be used include transfection reagents, which are used to increase the transfection efficiency of non-viral genetic delivery systems (e.g. RNA or plasmid DNA) into cell cultures. Transfection reagents include cationic lipids (such as e.g. Lipofectamine from Invitrogen) and cationic polymers (such as e.g. polyethylenimine (PEI) and derivatives, polylysine and derivatives, chitosan and other amino containing sugars, or polyamidoamine dendrimers). These may form complexes including the nucleic acid to be transfected. Transfection reagents such as PEI and lipofectamine are widely available commercially and may conveniently be used in the context of the present invention. Other additives that may be used include additives that bind or stabilise viral particles, such as viral receptors or sucrose. Additives including transfection reagents may be included in a bioink composition as described herein by including the additive or the complex comprising the additive and the nucleic acid or viral particle in the bioink formulation or in one of the stock solutions used to prepare the bioink formulation. For example, DNA/RNA molecules complexed with a transformation agent such as PEI or lipofectamine may be included in a stock solution of gelatin, that may then be used to prepare a bioink as described herein. Some biomaterials that are capable of forming a hydrogel can also be modified to assist with one or more aspects of transformation, for example binding viral particles, complexing naked DNA, improving transformation efficiency, etc. Such transformations may involve the chemical modification of biomaterials capable of forming a hydrogel, for example by addition of positively charged groups or compounds (e.g. amino groups). Examples of modified biomaterials capable of forming a hydrogel include cationic gelatin and cationic alginate. Cationic gelatin and alginate can be obtained by treating a solution of gelatin / alginate with diaminohexane. Suitable protocols for this are known in the art. Further, protocols usable for this purpose are provided in the Examples. For example, Padmanabhan and Smith (Pharm Dev Technol. 2002 Jan;7(1):97-101) have shown that modified alginate could be used to encapsulate plasmid DNA in order to transfect adherent cell cultures. The present inventors have shown that cationised alginate could be used as part of a composition used to form a hydrogel, in which both cells and a transforming agent are included, and that this may enable the transformation of cells growing within the hydrogel. The use of cationic gelatin as a part of an *in vivo* gene delivery system was investigated by Thakor et al. (Mol. Ther. (2007) 15(12), 2124-2131). The present inventors have shown that cationised gelatin could be used as part of a composition used to form a hydrogel, in which both cells and a transforming agent are included, and that this may enable the transformation of cells growing within the hydrogel. Thus, in the context of the present disclosure, a cationic bioink formulation may be used to bind viral particles, improve the stability or bioavailability of non-viral vectors, etc. The concentration of additives that may be used depends on the additive and the conditions of use. Commercially available additives may be used by mixing a solution comprising a transformation agent with one or more additives according to manufacturer instruction. Modified materials capable of forming a hydrogel may be used instead of the corresponding default material (e.g. cationic gelatin may be used instead of gelatin in the same proportions as mentioned above for gelatin). Further, it is also possible for transformation efficiency within the 3D hydrogel matrix to be enhanced by applying physical means such as sonoporation or electroporation. Sonoporation (also known as cellular sonication) and electroporation are commonly used in the art to induce cell membrane permeabilization thereby increasing transformation efficiency in suspension cultures. These technologies have also been used in *in vitro* tissue models and *in vivo*. Similar techniques and technologies can be used in the context of the present invention. Further, sonoporation and electroporation can both be applied in a localised manner.

A cellularised object according to the invention is preferably maintained within a liquid culture medium, wherein the cellularised object is fully immersed within the medium. The culture medium (also referred to simply as "medium") can be any medium that is suitable for the culture of cells as described herein, in conventional stirred bioreactors. In embodiments, the culture medium is a serum free culture medium, or a chemically defined culture medium. In embodiments, the culture medium does not comprise additives used to protect the cells from hydrodynamic damage, such as detergents. The culture medium may be the same culture medium used to prepare the bioink. However, this is not necessarily the case. For example DMEM(-) may be used to prepare the bioink, and DMEM (containing calcium) may be used for the culture of the bioprinted construct.

As used herein, a "transforming agent" (also referred to herein as "transformation agent") refers to a compound, complex or structure comprising nucleic acid suitable for introduction of said nucleic acid into cells. The transforming agent may be or comprise a DNA molecule such as a plasmid, an RNA molecule such as a small interfering RNA (siRNA) or microRNA (miRNA), a virus (also referred to as "virion", "viral particle" or "viral vector" - the latter being primarily used when the virus is engineered to introduce a nucleic acid into the cell that is not part of the normal genetic code of the virus), or any other DNA or RNA containing complex or structure. For example, a transforming agent may be a complex comprising a transfection reagent and a nucleic acid, such as e.g. a PEI-plasmid DNA complex. As another example, a transforming agent may be a viral particle comprising viral DNA or RNA and a viral capside. The process of introducing a transforming agent into a cell may be referred to as "transformation". The transformation process typically results in a cell whose pattern of gene expression is modified in some way, for example through expression of a transgene encoded by the nucleic acid introduced into the cell, through production of further viral particles by the expression machinery of the cell under the control of viral DNA present in a virus introduced in the cell, or through modified expression of an endogenous gene as a consequence of the presence of the exogenous nucleic acid into the cell. Such as cell may be referred to as an "engineered cell". The transformation process may be referred to as transfection if the introduction of the nucleic acid into the cell relies on a non-viral method, and transduction is the process relies on a virus.

**Figure 1C** illustrates schematically a process for the production of cellular products (engineered cells and products thereof) according to embodiments of the invention. A deposited object 20 is provided, in which cells 24 are embedded. The deposited object 20 also comprises embedded transforming agent 25. In the illustrated embodiment, the transforming agent is a viral vector 25B or a DNA complex 25A comprising a transformation additive 25A' and a plasmid 25A". The deposited object is cultured in liquid medium in a vessel (not shown), as indicated by arrow 50. As the cells grow within the hydrogel matrix, they enter into contact with the transforming agent 25, resulting in the formation of transformed cells. In the embodiment shown on the top row, the cells 24 and transforming agent 25 are embedded throughout the deposited object. This can be achieved for example by using one bioink composition to generate the deposited object, the bioink composition comprising both the cells and the transforming agent. In the embodiment shown on the bottom row, the cells 24 are initially embedded in a first region 20A of the deposited object and the transforming agent 25 is embedded in a second region 20B of the deposited object. The cells 24 that grow into the second region 20B from the first region 20A enter into contact with the transforming agent 25 and become transformed cells. This can be achieved for example by using two bioink compositions to generate the deposited object, one comprising the cells and another one comprising the transforming agent. The two compositions may comprise the same biomaterials capable of forming a hydrogel. Alternatively, the two compositions may comprise different biomaterials capable of forming a hydrogel. For example, the composition used to form the second region 20B may comprise one or more modified biomaterials capable of forming a hydrogel, for the purpose of stabilising the transforming agent and/or increasing transformation efficiency. The composition used to form the first region 20A may comprise different biomaterials or non-modified versions of the same biomaterials. For example, region 20A may be formed using a bioink comprising fibrinogen, gelatin and alginate (and cells), and region 20B may be formed using a bioink comprising fibrinogen, cationised gelatin and/or alginate (and one or more transforming agents). Either or both solutions may further comprise one or more additives. **Figures 1A and 1B** illustrate schematically comparative processes. In the process on Figure 1A, transformation occurs on a solid scaffold or in a 2D culture, by diffusion / sedimentation of DNA-transfectant complexes. In the process on Figure 1B, reverse transformation occurs by migration of seeded cells in a hydrogel matrix. This process is referred to as "reverse transformation" because the transforming agent is fixed / deposited in a matrix or on a surface, and the cells are then seeded onto the matrix / surface. An example of such a system is described in Sapet et al. (Ther. Deliv. (2013) 4(6), 673-685) where a thin layer of hydrogel comprising DNA/transfection complexes is provided, onto which cells are seeded. The cells migrate into the hydrogel where they are transfected. Thus, transfection happens on a relatively short term (24 to 72 hours) and not alongside cell growth. This in terms makes it poorly suitable for the industrial production of transformed cells or their products, as the amount of cells transformed is low, the system does not support substantial cell growth necessary to obtain industrial amounts of cellular products, and transformation may have to be repeated if the transformed cells are harvested and expanded and the transforming agent is not propagated to daughter cells.

**Figure 2A** illustrates schematically an exemplary system according to the disclosure. The system comprises a vessel (also referred to as "bioreactor") 10, in which one or more deposited objects (also referred to as "cellularised structure") 20 is/are maintained in a culture medium 30. In the illustrated embodiment the culture medium 30 completely surrounds the deposited object(s). In the illustrated embodiment, the deposited object(s) is/are each supported by an internal surface 12 of the bioreactor 10. In other embodiments, the deposited object(s) may be in suspension in the culture medium 30. In such embodiments, an agitation system (e.g. a stirring system) may be provided. In the illustrated embodiment, a single deposited object is shown, supported by an internal surface 12 of the bioreactor 10. However, in alternative embodiments a plurality of deposited objects may be supported by the same or different internal surfaces of the bioreactor and/or may be in suspension in the bioreactor. The internal surface(s) may be an internal wall that is part of the outer structure of the bioreactor, or may be an additional wall that is provided at least in part for the purpose of supporting the one or more deposited objects. For example, the bioreactor may be provided with one or more shelves each supporting one or more deposited objects. Optionally, the bioreactor 10 has one or more inlets 14 and one or more outlets 16 to allow the flow of culture medium 30 into and out of the bioreactor 10. In other words, the system may be a perfusion system (i.e. the vessel may be a perfusion bioreactor). A perfusion bioreactor is a cell culture vessel in which cells are retained in the vessel while a continuous inflow of fresh culture medium is provided and a continuous outflow of spend medium is extracted. The present inventors have discovered that a perfusion system enabled more efficient growth of cells at least partially embedded within a 3D hydrogel. The inlets 14 may be connected to a supply system (not shown), which may include e.g. a tank. The tank may be a stirred tank. The outlets 16 may be connected to a collection system (not shown), which may include one or more tanks, filtration systems, etc. The deposited object 20 comprises a matrix 22, cells 24 at least partially embedded in the matrix, and one or more transforming agent(s) 25 embedded in the matrix. In the illustrated embodiment, the matrix 22 is arranged in a three-dimensional structure that forms cavities or pores 23. As the deposited object 20 is maintained in the bioreactor, the cells may grow to colonise the pores 23 within the deposited object 20. In the illustrated embodiment, the deposited object 20 comprises a plurality of regions 20A comprising cells 24 at least partially embedded in the matrix 22, and a plurality of regions 20B comprising a transforming agent 25 embedded in the matrix 22. In the embodiment shown, the regions 20A and 20B are shown as alternating layers. However, any configuration of regions are envisaged, such as e.g. concentric regions (as illustrated e.g. on Figure 15), laterally adjacent regions (as illustrated e.g. on Figure 1C), dispersed regions (e.g. localised regions 20A or 20B surrounded by regions 20B or 20A, respectively), etc. Thus, the invention encompasses in particular any arrangement obtained by separately providing a bioink comprising cells and a bioink comprising a transforming agent, to form a single deposited object comprising spatially distinct regions. In other words, the invention encompasses any configuration of deposited object comprising one or more regions in which cells are at least partially embedded in a 3D hydrogel matrix and one or more regions in which transforming agents are embedded in a 3D matrix hydrogel, the regions being adjacent to each other.

**Figure 2B** shows a simplified process diagram for a generic bioprocess according to the disclosure. The illustrated process comprises optional step 220 of providing one or more bioink compositions comprising one or more biomaterials capable of forming a hydrogel. When a single bioink composition is provided, the composition comprises both cells and one or more transforming agents. Alternatively, separate bioink compositions may be provided, comprising either cells or transforming agents. Preferably, the separate bioink compositions comprise the same or similar biomaterials capable of forming a hydrogel. The term "similar" in relation to biomaterials refers to biomaterials that are capable of forming a hydrogel by cross-linking with each other. For example, gelatin and cationised gelatin may be considered to be similar materials. The term "similar" in relation to a bioink composition refers to bioink compositions comprising biomaterials that can be consolidated using the same protocol (e.g. the same consolidation solution). For example, a bioink composition comprising fibrinogen, alginate and gelatin is similar to a bioink composition comprising fibrinogen, alginate and cationised gelatin. Indeed, it contains similar materials and can be consolidated using the same protocol. Similarly, a bioink composition comprising fibrinogen, alginate and gelatin is similar to a bioink composition comprising fibrinogen, gelatin and cationised alginate. The use of the same (or similar / corresponding) biomaterials may ensure that the same consolidation protocol can be applied to both bioink compositions, and hence that the resulting consolidated object will comprise regions made from one bioink composition that are physically integrated with regions made from the other bioink composition (by cross-linking). In other words, the object comprises regions that are distinct in composition but that are not physically disjoint. The bioink composition may be deposited into a three-dimensional structure at step 230, thereby forming a deposited object / cellularised structure. This may be performed either by additive manufacturing, or by moulding. In embodiments where a plurality of bioink compositions are provided, these may be deposited together or separately. For example, deposition of two bioink compositions by additive manufacturing may be performed using a concentric nozzle, resulting in a filament comprising an external region made from a first bioink composition and a core region made from a second bioink composition. The same concept can be extended to three or more regions. Alternatively, deposition of two bioink compositions by additive manufacturing may be performed using two adjacent nozzles (or a nozzles comprising receiving two separate feeds, if the viscosity of the two compositions is such that the two feeds do not mix while in the nozzle). Again, the same concept can be extended to three or more regions. Similarly, the moulding of an object comprising regions made from different bioink compositions can be performed by simultaneously, subsequently or alternately depositing the different compositions in a mould (e.g. by pouring or in any other way). The bioink composition may then be consolidated at step 240. When a plurality of bioink compositions are provided and deposited separately, the deposited objects may be consolidated together or separately. When the deposited objects are consolidated separately, they may each be consolidated using a first consolidation composition, then brought into physical contact with each other and consolidated using a second consolidation composition. The second consolidation composition may comprise transglutaminase. The first consolidation composition may not comprise transglutaminase. The present inventors have identified that consolidation in two steps using transglutaminase in a second step to unify deposited objects that have been separately pre-consolidated resulted in integral deposited objects suitable for the growth of cells throughout the regions that have been separately consolidated. At step 250, the cellularised structure is cultured, resulting in the growth of the cell population. As the cell population grows, it enters into contact with the transforming agents, thereby forming a transformed cell population. At optional step 260, one or more cellular products of the transformed cell population are harvested. This may include the step of removing some or all of the culture medium (optionally with replacement, for example where the harvesting step is performed during the course of cell culture rather than at the end of it). Instead or in addition to this, harvesting a cellular product may comprise separating the cells from the hydrogel matrix, at the end of the culture. Embodiments of these generic steps are described in more detail by reference to Figure 3.

**Figure 3** illustrates schematically a process for production of cellular products according to embodiments of the present disclosure. At step 300, a population of cells (e.g. production cells, for example from a production cell line) is amplified using conventional cell culture protocols such as e.g. using a suspension culture or adherent culture. The choice of a suitable amplification protocol may depend on the cell type at hand. In parallel, a solution of transforming agents is also obtained, for example by expanding a plasmid population in a bacterial culture. At step 310, the amplified cell population is harvested, for example by centrifugation and/or trypsinisation (depending on the cell culture protocol used at step 300). In parallel, the transforming agent may be extracted from the expansion solution and purified, e.g. using plasmid extraction protocols known in the art. This may also comprise the step of incubating the transforming agent with one or more transformation additives, such as e.g. PEI. This may result in a solution of transformation agent-plasmid complex. At step 320 a bioink is formulated by mixing the harvested cells with a solution comprising biomaterials capable of forming a hydrogel (in particular, gelatin, alginate and fibrinogen, in the illustrated embodiment). The transforming agent (optionally including one or more additives) may be combined with one of the solutions used to formulate the bioink. For example, the transforming agent (optionally complexed) may be included in a solution comprising gelatin and/or alginate, which is then used to formulate the bioink at step 320. At step 330, the bioink composition is 3D printed, for example by pneumatic extrusion in the illustrated embodiment. At step 340, the bioink composition is consolidated. In the illustrated embodiment, the structure formed by the gelatin is at least partially dissolved and a more permanent structure is formed by cross linking the alginate and fibrinogen, respectively. The partial dissolution of the gelatin may create microporosities that advantageously enable further cell growth. When transglutaminase is used in the consolidation process, the gelatin may be stabilised by cross-linking and hence dissolution of the gelatin may be reduced or absent. This may help with the stabilisation of the transforming agent in the deposited object. A balance between the creation of microporosites by dissolution of the gelatin and the stabilisation of a transforming agent which is enhanced by the presence of gelatin may be obtained by adjusting the degree of cross-linking of the gelatin. This may be achieved by adjusting the parameters of the consolidation with transglutaminase (such as e.g. by adjusting the incubation time and/or concentration of transglutaminase). At step 350, the cellularised structure is cultured in a culture medium, during which the cell population grows to colonise the structure. In this process, at least some of the cells enter into contact with transforming agents and become transformed cells. This may happen continuously, as cells grow to reach more transforming agent. At step 360, the cells are harvested by dissociating the hydrogel matrix. The cells may be separated between transformed cells and non-transformed cells, for example on the basis of expression of one or more markers encoded by the transforming agent. For example, cells expressing one of more fluorescent markers encoded by the transforming agents may be separated by flow cytometry.

**Figure 4** illustrates schematically a process for providing a cellularised structure according to embodiments of the disclosure. At step 420A, a solution of cells (such as e.g. cells suspended in culture medium or buffer, preferably calcium free culture medium such as DMEM(-)) is mixed with stock solutions respectively comprising 5 % w/v gelatin, 2 % w/v alginate and 2 % w/v fibrinogen. At step 420B, a solution of transforming agent (such as e.g. plasmids -optionally complexed e.g. with PEI, in distilled water or culture medium, or virus particles) is mixed with stock solutions respectively comprising 5 % w/v gelatin, 2 % w/v alginate and 2 % w/v fibrinogen. Alternatively, one of the stock solutions may include the transforming agent. For example, the stock solution of gelatin may be obtained using a solution comprising the transforming agent, and gelatin. Preferably, each of these stock solutions are prepared in culture medium, which is typically calcium free. Note that alternative concentrations of each of these components and compositions comprising two or all three of these biomaterials may be used instead. The solutions obtained at steps 420A/420B may be referred to as a bioink. At step 425A, the bioinks are incubated at 37C to allow the solution to homogenise and the cells to recuperate, for example for 10 minutes. This step may be omitted for the bioink composition not comprising cells, and/or may be performed for the stock solutions prior to preparation of the bioink composition rather than after preparation of the stock solutions. At step 425B, the bioinks are incubated at 21C to allow the compositions to reach a viscosity suitable for printing, for example for 30 minutes. This may be performed in the same syringe that will be used for printing. Thus, depending on the printing configuration this may be performed separately for the two bioink compositions, or within the same device. Note that the precise times and temperatures provided are indicative only, and alternative times / temperatures may be used depending on the cell population, the composition of the bioink (ingredients and concentrations) and the desired printing viscosity (which can depend on the printing parameters). At step 430, the bioinks are printed into a predetermined 3D structure. At step 440, the deposited object is consolidated by incubation with a consolidation solution. The timing of consolidation may depend on the concentration of the consolidation solution and the size of the object. Indicative times are provided. The temperature of consolidation may depend on the cells and on the composition of the bioink (for example, the temperature may be chosen to be sufficient to at least partially re-melt the gelatin without damaging the cells). At step 450, the consolidated cellularised structure is cultured in a bioreactor.

_Examples_

Exemplary methods of transforming cells within a 3D hydrogel matrix, as well as exemplary methods for obtaining a transforming and proliferative cell culture in a 3D hydrogel matrix will now be described.

_Materials and Methods_

Generally, cells were amplified in "traditional" culture in suspension or as adherent cultures in flask (depending on the cell type) (Fig. 3, step 300). The cells were then trypsinised (Fig. 3, Step 310) if grown as adherent cultures, and formulated as a bioink formulation (Fig. 3, step 320). The formulation was 3D printed (Fig. 3 step 230), and the construct was consolidated (Fig. 3, step 340). After culture (Fig. 3, step 350), the cellularised structures can be dissociated to harvest the cells (Fig. 3, step 360) if desired.

_Cell trypsinisation - HEK293 cell line._ HEK293 cells were cultivated in T75 flasks (Corning). The culture media was carefully discarded from the flasks and cells were gently rinsed with 5 mL PBS 1X (Gibco). PBS was removed from the flasks and 5mL of DMEM without calcium DMEM (-) (Gibco) was added in the flasks. Cells were detached from the flask by gently shaking them. The detached cells in DMEM (-) were recovered in a 50ml falcon tube and counted on an hematocymeter for cell concentration determination.

_Cell trypsinisation - MDCK cell line._ MDCK cells were prepared in T75 flasks (corning). The culture media was carefully discarded from the flasks and cells were gently rinsed with 5 mL PBS 1X (Gibco). PBS was removed and 10 mL of fresh PBS were added. The flasks were then incubated 40 min to 1h at 37°C. The PBS was then removed and 5 mL of trypsin-EDTA 1X (Trypsin : 0.25 g/L, 0.0105mM ; EDTA 4Na 2H2O : 0.038 g/L, 0.09130226 mM) (GibcoTM, Trypsin-EDTA (0.5%), no phenol red, 15400054) were added in each flask and incubated 2 to 5 min at 37°C to completely detach the cells. After tapping the flasks, 10 mL of DMEM(-) (Gibco) were added in each flask to inactivate the trypsin. The detached cells in DMEM(-) (Gibco) were recovered in a falcon 50mL tube and counted on an hematocymeter.

_Cell trypsinisation - CHO cell line._ CHO cells were cultivated in suspension in T75 flasks (Corning).

The culture medium with the suspended cells was recovered in a 50ml falcon and centrifugated for 5 min at 300g. The bottom of the flask was rinsed with PBS 1X (Gibco) and 2 mL trypsin-EDTA 1X (Trypsin : 0.25 g/L, 0.0105mM ; EDTA 4Na 2H2O : 0.038 g/L, 0.09130226 mM) (GibcoTM, Trypsin-EDTA (0.5%), no phenol red, 15400054). were then added to detach the adhering cells. The flask was incubated 2 min at 37°C and then 5 mL of DMEM(-) (Gibco) was added to the flasks to inactivate trypsin. The detached cells were recovered and added to the tube containing the media with suspension cells. The cells were counted on an hematocymeter.

*Cell trypsinisation - VERO cell line.* VERO cells are cultivated in T75 flasks (Corning). The culture media was carefully discarded from the flasks and cells were gently rinsed with 5 mL PBS. PBS was removed prior addition of 2.5 mL of trypsin-EDTA 1X (Trypsin : 0.25 g/L, 0.0105mM ; EDTA 4Na 2H2O : 0.038 g/L, 0.09130226 mM) (GibcoTM, Trypsin-EDTA (0.5%), no phenol red, 15400054) were added in each flask and incubated 2 min at 37°C to completely detach the cells. After tapping the flasks, 5 mL of DMEM(-) were added in each flask to inactivate the trypsin. The detached cells in DMEM(-) were recovered in a falcon 50mL tube and counted on an hematocymeter.

*Stock solutions for bioink formulation.* Stock solutions were prepared the day prior bioprinting to allow for best component dissolution. Fibrinogen stock solution was prepared by dissolving 8% w/v Fibrinogen from bovine plasma (F8630-1G, Merck) in DMEM without calcium (Gibco). Alginate stock solution was prepared by dissolving 4% w/v Alginate (Alginic acid sodium salt very low viscosity, A18565.36, Alfa Aesar) in DMEM without calcium (Gibco). Gelatin stock solution was prepared was prepared by dissolving 20%w/v Gelatin from porcin skin (Sigma Aldrich, G1890) in DMEM without calcium (Gibco).

Plasmids stock preparation were performed using an *E. coli* strain containing the plasmid carrying reporter genes (pEGFP-C1 or pmCherry-C1). Bacteria were cultured overnight (15 to 24h) in a Luria Broth medium (Sigma - L3022-1KG). Bacteria were recovered by centrifugation 15 min at 3220 g. Plasmids were extracted using the Qiagen Plasmid Maxi Kit (10) ref : 12162) and resuspended in sterile deionized water. Plasmid were stored at concentration of 1.4 µg/mL and 1.5 µg/mL at -20°C prior use.

*Bioink formulation.* See Fig. 4, steps 420, 425A, 425B. After cell harvesting (trypsinization of adherent cells - see above, or centrifugation of suspension cells), targeted cell concentration is adjusted by pelleting the appropriate volume of cells at 300g during 5 min. For the transformation experiments (Examples 3-5), preferred concentrations ranged from $1 \times 10^6$ to $3 \times 10^6$ cells/mL of biomaterial. The pellet is resuspended in the appropriate volume of Fibrinogen 8% solution. The appropriate volumes of Alginate 4% solution and the Gelatin 20% solution are further added with a final proportion 25% Fibrinogen solution, 25% Alginate solution and 50% Gelatin solution corresponding to a final concentration in the bioink of 2%w/v Fibrinogen, 2%w/v Alginate, and 5%w/v Gelatin (Fig. 4, step 420). This formulation ensures a rheofluidifying behavior of the bioink ensuring the protection of cells while printing. The cells and the three components of the bioink were mixed and homogenized by carefully pipetting 10 times with a viscous-liquid pipet. The bioink was then incubated 10 min at 37°C (Fig. 4, step 425A) before being poured into a printing sterile syringe. The bioink is then incubated 30 min at room temperature (21°C) before being used (Fig. 3, Step 425B) for cellularised structure 3D bioprinting. When plasmids or viruses were included in the bioink formulation, they were added to the gelatin 20% solution and incubated either overnight or 10 min, at 37°C before the preparation of the cellularized biomaterial. In particular, the plasmids used were extracted from E.coli strains, purified and suspended in sterile deionized water for storage at -20°C. The adequate volume of plasmid in sterile deionized water was then pipetted and added directly to the gelatin solution. The concentration of the plasmid/virus in the gelatin stock solution was adjusted to obtain the desired final concentration in the formulated biomaterial.

*Bioink formulation with additives and transforming agents.* Additives are molecules that are enhancing the potential of nucleic acids (DNA or RNA) to penetrate in the targeted cells. As an example, the plasmids were complexed with a transfection reagent (PEI or Lipofectamine 3000) according to the supplier recommendations. PEI (Polysciences Inc - 23966-1)/DNA complexes were prepared by mixing DNA with PEI at w/w ratios 2:1 (PEI:plasmid) in DMEM without calcium. Lipofectamine3000 (InvitroGen - L3000-001)/DNA complexes were prepared by mixing DNA with PEI at v/w ratios 1:1:1 (Lipofectamine P3000 : plasmid) in DMEM without calcium. The complexed plasmids were added to the gelatin 20% solution and incubated either overnight or 10 min, at 37°C before the preparation of the cellularized biomaterial. No difference in stability of the complexed plasmids or transfection efficiency was observed depending on the incubation time (overnight vs 10 min). The use of a 37°C incubation temperature advantageously ensures that the gelatin is fluid enough to enable a good mixing of the components. The plasmid concentration within gelatin should be adjusted according to the final concentration expected in the formulated biomaterial where gelatin represents 25%v/v. As an example, final complexed plasmids concentrations tested were of 4µg/mL or 10 µg/mL.

*Bioink formulation with modified biomaterial components.* To prepare a transfecting cationic biomaterial, the plasmids were added at concentrations ranging from 40 µg/mL to 68 µg/mL in the cationic gelatin to reach final concentrations from 10µg/mL to 17 µg/mL in the biomaterial where gelatin represents 25%v/v. Thus, the plasmid concentration within gelatin should be adjusted according to the final concentration expected in the formulated biomaterial. As an example, plasmids were added to the cationic gelatin 20%w/v solution and incubated 30 min, at 37°C before the preparation of the bioink. As mentioned above, no difference in stability of the complexed plasmids or transfection efficiency was observed depending on the incubation time (overnight vs 10 min) when using transfection reagents. Thus, shorter incubation times are believed to be sufficient. Nevertheless, slightly longer incubation times such as e.g. 30 minutes may enable improved complexation of the DNA with some modified biomaterials such as cationised gelatin. The use of a 37°C incubation temperature advantageously ensures that the gelatin is fluid enough to enable a good mixing of the components, and thus good complexation of the plasmids with cationised gelatin. Negative controls were prepared with standard biomaterial formulation and cationic biomaterial, both prepared without addition of plasmids. A comparative condition was also prepared with standard bioink containing naked plasmid (see below). Transfection was monitored by fluorescence microscopy observations or dissociation of the tissues and flow cytometry on the recovered cells. A similar protocol may be used with cationised alginate (i.e. including the plasmids in the solutions comprising cationised alginate instead of the solution comprising cationised gelatin).

*Production of cationized gelatin.* Cationic gelatin was prepared by dissolving gelatin (Sigma Aldrich - G1890-500G) in a pH 5.8 phosphate buffer (40 g/L). After complete dissociation at 37°C, diaminohexane (Fluka - 33000) was added for a concentration of 1.16 M. After complete dissolution, the pH was adjusted to pH5 by addition of highly concentrated HCL (37%). An important volume of HCl is necessary to reach pH5. EDC (Ethyl-3-(3-dimethylaminopropyl) Carbodiimide) (Sigma Aldrich - E7750) was added to the solution to a final concentration of 0.116 M. The vessel was then covered with plastic film to avoid evaporation and the solution vas incubated 18h to 24h at room temperature with agitation. The solution was then placed in dialysis pouches with extrusion size 500 -1000 Dalton (Interchim - Float-A-lyser - G235063) and dialysed 48h at room temperature against deionized water. The dialysate was then recovered, lyophilized during 72h and the lyophilized powder was stocked at -80°C.

*Production of cationized alginate.* Alginate powder (Alfa Aesar - A18565) was dissolved at a concentration of 1g/L in a 2-[N-Morpholino] ethanesulfonic acid (MES) 0.1M and NaCl a 0.5M (pH 4.5) buffer. EDC (Ethyl-3-(3-dimethylaminopropyl) Carbodiimide) (Sigma Aldrich - E7750) was added to the solution for a final concentration of 5g/L. Diaminohexane (Fluka - 33000) was then added for a final concentration of 9.3 mM. The preparation vessel is covered to avoid evaporation and the solution was incubated 2h at room temperature under agitation. The solution was then placed in dialysis bags with exclusion size 3500 -5000 Dalton (Interchim - Float-A-lyser - G235065) and dialysed 24h at room tempera-

ture with 0.9% NaCl in deionized water solution. The dialysate was then recovered, lyophilized during 72h and the lyophilized powder was stocked at -80°C.

*Bioink rheology.* The appropriate rheology of the bioink for printing of in particular large structures (e.g. above 10 cm$^3$) with porosity can be established by comparing a rheogram acquired for each composition with a standard (reference) rheogram for a bioink composition with known rheological properties (see **Figure 15A**). For a composition with these properties, a range of temperature compatible with printing, where the properties of the composition are such that high cell viability is maintained while allowing to maintain high fidelity of the deposited shape, was determined. This was determined by determining the range of values of maximum shear stress and static yield stress that are compatible with maintaining the shape fidelity of the bioprinted object, and the range of values of maximum shear stress and static yield stress that are compatible with maintaining the viability of the cells after microextrusion (assessed using cell viability of NIH fibroblast cells included in the bioink). The results of this for an exemplary bioink composition are shown on **Figure 15B,** where the shaded zone represents the shear stress (MSS) and static yield stress (SYS) that cells can handle (NIH viability limit) and that also provides suitable shape fidelity (STL fidelity). Based on the data on Figure 15B, a printing temperature between 21 and 28°C was chosen. The shear rate on the wall of the extrusion nozzle ($\dot{\gamma}_w$) was calculated in order to determine the shear stress experienced by the cells in the printing nozzle. To obtain the wall shear stress value, the shear rate in the syringe nozzle and the associated viscosity were independently determined. First, a stress controlled rotational rheometer (DHR2, TA instruments) was used to measure the static yield stress of bioink and the shear stress felt by cells during the bioprinting process. A plan-plan (40 mm) geometry was used with a shear rate sweep procedure at different temperatures (21, 23, 28 and 37°C). From this experiment, bioink shear stress was determined over a large shear rate scale [0.01; 100] s$^{-1}$.

Second, the wall shear rate in the cartridge was defined from a traditional Poiseuille equation as a function of flow (equation 1):

$$\gamma_w^{t,b} = \frac{3n+1}{n} \frac{Q}{\pi R_{t,b}^3} \quad (1)$$

where n is the flow behavior index determined in the rotational rheological experiment, Q (mm$^3$.s$^{-1}$) is the flow and $R_{t,b}$ (mm) is the radius at the top (t) and bottom (b) of syringe nozzle. A flow value of 0.183 mm$^3$.s$^{-1}$ was used in these experiments. As the printing nozzle used was frustoconical, the wall shear stress increases from the top to the bottom of frustoconical printing nozzle and results were therefore obtained for both the top and bottom of the nozzle.

*Formulation and culture - HEK293 cell line.* Experiments were carried out with HEK293 cells seeded at different cell densities, these cell densities were ranging from $1 \times 10^6$ cells/ mL to $10 \times 10^6$ cells/ mL of bioink. Cellularised square structures of 0.2cm$^3$ or tubes were cultivated in 2mL culture medium.

*Formulation and culture - MDCK cell line.* Experiments were carried out with MDCK cells seeded at a density of $1 \times 10^6$ cells/ mL of bioink and $3 \times 10^6$ cells/ mL of bioink. Tissues of 0.2cm$^3$ were cultivated in 2mL culture media corresponding respectively to cell concentrations of $1 \times 10^5$ cells/ mL of medium and $3 \times 10^5$ cells/ mL of medium.

*Formulation and culture - CHO cell line.* Experiments were carried out with CHO cells seeded at a density of $1 \times 10^6$ cells/ mL of bioink. Tissues of 0.2cm$^3$ were cultivated in 2mL culture media corresponding to a cell concentration of $1 \times 10^5$ cells/ mL of medium.

*Formulation and culture - VERO cell line.* Experiments were carried out with VERO cells seeded at a density of $3 \times 10^6$ cells/ mL of bioink. Tissues of 0.2cm$^3$ were cultivated in 2mL culture media corresponding to a cell concentration of $3 \times 10^5$ cells/mL of medium.

*3D printing.* See Fig. 4, step 430. The printed structures were designed by Computer Assisted Design (CAD) using 3D modelling software like Autodesk® Fusion 360™ and 3D Builder (Microsoft Corporation). 3D CAD files were converted to .stl format to be transferred to 3D printer equipment. The cellularized structure were bioprinted by pneumatic micro-extrusion using a Bio-AssemblyBot 3D printer (Advanced Solutions, Inc). Inket technologies can be used instead of micro-extrusion. The bioink was prepared as described above and placed within printing syringes of 10cc or 30cc according to the volume of bioink prepared.

The needles used with these syringes had diameters ranging from 200 $\mu$m to 800 $\mu$m diameter. The printing process was carried out at 21°C with pneumatic pressures ranging from 20 to 50 psi. These ranges were found to result in the formation of a smooth continuous filament.

*Consolidation.* See Fig. 4, step 440. The bioprinted structures were consolidated with one of multiple alternative solutions comprising one or more of the following ingredients: CaCl$_2$ (C5670-500G, Merck), transglutaminase (TAG) (ACTIVA WM, Ajinomoto) and thrombin (Thrombin from bovin plasma, t4648-10KU, Merck) in deionized water. Unless indicated otherwise, all three ingredients were used. Preferred concentrations are 3% w/v CaCl2, 4% transglutaminase and 10 U/mL of Thrombin. The consolidation process was carried out at 37°C for 1 hour for the small tissues of 0.2 cm$^3$ and for 2 hours for large tissues above 10 cm$^3$. The printed consolidated structures were then rinsed twice with sterile physiological serum at a ratio of 10 mL physiological serum for 1mL of bioprinted material.

*Cell harvesting by dissociation with collagenase A 3% In PBS (phosphate-buffer saline).* A collagenase A (COLLA-RO Roche) 3% w/v (4.5 U/mL) was prepared in PBS 1X (Gibco) solution and warmed at 37°C in a water bath. The tissues were rinsed once with 2mL PBS 1X (Gibco), then weighted before being immersed in the collagenase solution (0.5 mL for a 0.2 cm$^3$ tissue). Cellularised structures were then incubated at 37°C in a water bath with vigorous agitation every 10 to 20 minutes until complete dissociation (-40 min - 1h for a 0.2 cm$^3$ tissue). The tubes were centrifuged and the cell pellets were resuspended in 1mL PBS.

*Growth and expression monitoring.* After bioprinted structure consolidation, cultivation was performed in culture well plates with appropriate culture media. Monitoring of the transformation of the embedded cells within the biomaterial was performed by monitoring daily the apparition of reporter gene fluorescence expression by fluorescence microscopy. Quantification of the cell population transformed was realized thanks to biomaterial dissociation (using a dissociation with collagenase A (COLLA-RO Roche) 3% w/v (4.5 U/mL) in PBS 1X) and cytometry analysis. Media were changed 3 times a week (Monday, wesdnesday, Friday). The media used were: DMEM 1X (10566016 Gibco) supplemented with 10 % FBS for HEK, VERO and CHO experiments, VP-SFM (11681020 Gibco) was used for VERO cells and Sf-900 II medium for SF9 cells (10902096 Gibco).

*Growth monitoring by spheroid measurement.* For the cells growing in spheroids, microscopic pictures of bioprinted structures were taken. Using microscope scale bars, pictures were analyzed using the software ImageJ to measure spheroids diameters of at least 6 to 10 spheroids (due to a high variability in spheroids diameters).

*Evaluation of nucleic acid stability within the biomaterial.* DNA stability was evaluated thanks to labelling with nucleic acid intercalating dye, DAPI, which is fluorescent at 450-490nm. DNA was incubated with DAPI (11mM) prior formulation with the biomaterial at a concentration of 0.75$\mu$g/$\mu$l. Biomaterial was then consolidated as indicated above. DNA fluorescence was monitored over 15 days to confirm its stability within the biomaterial.

### Example 1 - Culture of production cell lines in 3D printed structures

In this example, a plurality of cell lines was cultured embedded in 3D printed hydrogel structures, in order to investigate the proliferation capacity within this context.

The results for CHO cells are shown on **Figure 5.** Cells were seeded at $1 \times 10^6$ cell/mL of bioink (eq. to $1.0 \times 10^5$ cell/ mL of medium) within a culture medium composed of DMEM and FBS (10%). The data shows that cell concentrations of about $2 \times 10^5$ cells/ml are reached after 20 days of culture (square data points), without any optimization, and that the density of live cells increases during culture, illustrating strong potential for growth, and long term viability in culture.

The results for HEK cells are shown on **Figure 6.** Cells were seeded at $1 \times 10^6$ cell/mL of bioink (eq. to

1.0 × $10^5$ cell/ mL of medium) within a culture medium composed of DMEM and FBS (10%). The data shows that cell concentrations of about 6×$10^5$ cells/ml are reached after 20 days of culture (square data points), without any optimization, and that the density of live cells increases during culture, illustrating strong potential for growth, and long term viability in culture.

The results for MDCK cells are shown on **Figure 7**. Cells were seeded at 1 × $10^6$ cell/mL of bioink (eq. to 1.0 × $10^5$ cell/ mL of medium) within a culture medium composed of DMEM and FBS (10%). The data shows that cell **concentrations** of about 3×$10^5$ cells/ml are reached after 20 days of culture (square data points), without any optimization, and that the density of live cells increases during culture, illustrating strong potential for growth, and long term viability in culture.

The results for VERO cells are shown on **Figure 8**. Cells were seeded at 3 × $10^6$ cell/mL of bioink within a VP-SFM (1X) culture medium (11681020, Gibco). The data shows that cell concentrations of about 3×$10^5$ cells/ml are reached after 20 days of culture (square data points), without any optimization, and that the density of live cells increases during culture, illustrating strong potential for growth, and long term viability in culture.

Thus, this data demonstrates strong proliferation of a variety of cells embedded in a 3D hydrogel matrix. This indicates that transformation triggered by and/or alongside growth of the cells may be possible in this context.

### Example 2 - Stability of transforming agent in 3D hydrogel matrix

In this example, the stability of transforming agents (plasmids and viral particles) in a 3D hydrogel matrix was investigated. The results of this are shown on **Figure 9** (plasmid) and **Figure 14** (virus). **Figure 11** shows transformation by transduction of HEK293 mammalian cells with GFP-lentivirus embedded in the proliferative biomaterial during cell growth. Cultivation is lasting for over 15 days. HEK293 cells 3D printed at cell density of 3 × 106 cell/ml with GFP-lentivirus.

In particular, the impact of consolidation solutions on the stability of plasmid DNA within the biomaterial was investigated by including naked plasmid in the stock gelatin solution used to prepare the bioink. Droplets of bioink were then produced and consolidated using different consolidation solutions (CaCl2 3%; TAG : Transglutaminase 4%; Throm : Thrombin 10U/ml). The plasmid DNA was labelled with DAPI and observed at 460 nm (excitation at 350 nm) over 15 days. The data on Figure 9 shows that plasmid DNA was stable in the consolidated bioink in all tested conditions. The data shown on Figure 9 was collected at day 13. Thus, the data on Figure 9 shows that all of the consolidation solutions tested allowed for long term stabilization of DNA in the bioink. The data on Figure 9 further indicates that the use of transglutaminase may allow to conserve a higher quantity of DNA compared to solutions without transglutaminase.

Further, the impact of consolidation solutions on the stability of viral particles within the biomaterial was investigated by quantifying after dissolution the biomaterial and quantifying viral particles thanks to Virocyt equipment. Infectivity was assessed thanks to standard infectivity assays, either TCID50, plaque forming units or transduction unit assays. The data on Figure 14 shows that viral vectors are present and can remain active in the bioink for over 17 days (i.e. long term transformation with a viral vector occurs within the bioink even after 17 days). Additional experiments will be performed to confirm the presence of infectious viral particles in the bioink after consolidation. These will include extracting virus back from consolidated biomaterial every 3 days for a duration of 21 days using dissociation protocols already evaluated to extract live cells (incubation with collagenase and alginate lyase). After extraction, bioactivity of the viral particles will be validated by applying the viral suspension on planar culture of sensitive cells (i.e. HEK293, Vero, MDCK cells) to observe either GFP expression (for viral vectors) or cytopathic effect (for pathogenic virus like influenza). In a second step, if the bioactivity is validated, quantification of the extracted virus will be performed to evaluate the yield of bioactive virus and its stability. This will be performed using standard TCID50 assays or Transduction Unit assays protocols. Such protocols consist in applying gradual dilution of the sample to test onto planar culture of sensitive cells to reach the limit dilution point allowing for calculation of the number of active particles present in the suspension. Based on the results already obtained, these experiments are expected to show that infectious viral articles can be maintained over long periods of time in the bioink.

This data shows that transforming agents can be included and stably maintained within a 3D hydrogel matrix.

### Example 3 - Long lasting transformation by transfection

In this example, the potential for long lasting transformation within the context of the 3D hydrogel matrix comprising both embedded cells and embedded plasmid was investigated. The results of this are shown on **Figures 10 and 12**.

In particular, HEK293 cells were cultured in a 3D hydrogel matrix (obtained by molding and consolidation) at a cell density of 3 × $10^6$ cell/ml with 17μg of GFP plasmid (pEGFP-C1) per milliliter of bioink. The bioink used was either a "standard" mixture of fibrinogen, alginate and gelatin (labelled "standard FAG") or a modified mixture comprising cationic gelatin instead of gelatin (labelled "FAG w/cationic gelatin"). The culture was observed by fluorescence microscopy on days 2, 6 and 8 (i.e. 2, 6 and 8 days after molding and consolidation), to monitor the presence of GFP which is indicative of successful transformation. The data on Figure 10 shows that successful transformation is observed at all times following consolidation, including in particular after 6 and 8 days, i.e. in conditions where transformation occurs in new generations of cells that have entered into contact with the transformant as they proliferated to colonize the hydrogel scaffold. The data further indicates that the use of cationic gelatin improves the transformation efficiency particularly after 8 days. Without wishing to be bound by theory, the inventors believe that this may be because the growth rate of the cells starts to increase around this time, leading to improved transfection. Indeed, proliferating cells are known to be more effectively transfected. After 2 days in culture in the bioink, the cells are likely still in a lag phase and not proliferating in the bioink. In other experiments, the inventors showed that cells usually start to grow faster around 10 to 15 days of culture. Thus, it is likely that after 8 days, the cells started to grow faster, allowing them to get in contact with the plasmids contained in the bioink and allowing the plasmids to enter the nucleus for efficient transfection. Figure 12 shows the results of a similar experiment where the standard mixture of fibrinogen, alginate and gelatin was used and GFP expression was monitored after 12 days in culture, as well as cell growth (optical microscopy) and cellular nuclei (DAPI fluorescence).

Further, HEK293 cells were cultured in a 3D hydrogel matrix (obtained by 3D printing and consolidation) at a cell density of $3 \times 10^6$ cell/ml with GFP-lentivirus embedded in a standard mixture of fibrinogen, alginate and gelatin. The culture was observed by fluorescence microscopy on days 2, 11 and 16 (i.e. 2, 11 and 16 days after printing and consolidation), to monitor the presence of GFP which is indicative of successful transformation. The data on Figure 11 shows that successful transformation is observed at least after a few days in culture, and that sustained expression is observed in long-term culture. As explained above, the delay in observing the transformation may be due to the presence of a lag phase in the growth of the cells, followed by an increase in growth rate positively impacting transformation efficiency. This indicates that transformation also occurs in new generations of cells that have entered into contact with the transformant as they proliferated to colonise the hydrogel scaffold.

### Example 4 - Effect of transformation additives

In this example, the potential effect of including transformation additives on transformation efficiency within the context of the 3D hydrogel matrix comprising both embedded cells and embedded plasmid was investigated.

In particular, HEK293 cells were cultured in a 3D hydrogel matrix (obtained by printing and consolidation) at a cell density of $3 \times 10^6$ cell/ml with 17μg of GFP plasmid (pEGFP-C1) per milliliter of bioink or the same amount of the same plasmid in complex with PEI. The printed constructs were dissociated with collagenase 3% in PBS. The expression of the transgene was observed by counting fluorescent cells after 3, 7 and 15 days in culture (3, 7 or 15 days after molding and consolidation) by flow cytometry. The data indicated that effective long-lasting transformation occurs in all conditions tested.

### Example 5 - Effect of transformation additives

In this example, a set up was investigated whereby cells are embedded within a 3D hydrogel matrix and a transforming agent is also embedded within the same matrix but in a different region of said matrix. The results of this are shown on Figure 14.

In particular, a bioink formulation comprising HEK293 cells was printed alongside a bioink formulation comprising GFP-lentivirus (both a standard fibrinogen, alginate, gelatin composition) using a set up as illustrated on **Figure 13.** As shown on Figure 15, the two bioink compositions were coextruded to produce a filament comprising a core comprising the transforming agent(s) and an outer region comprising the cells. The composition was printed then consolidated (with transglutaminase, thrombin and CaCl$_2$) and cultured for 17 days. The culture was observed by fluorescence microscopy on days 2, 6, 14 and 17 (i.e. 2, 6, 14 and 17 days after printing and consolidation), to monitor the presence of GFP which is indicative of successful transformation. The data on Figure 14 shows that such a set up enables successful transformation, and that the timing and location of transformation can be controlled by separating the transforming agent and cells in adjacent hydrogel regions. Indeed, as can be seen on Figure 14 only growing cells are transformed over time when they enter in contact with the transforming agent embedded and stabilized within the biomaterial.

### Equivalents and Scope

All documents mentioned in this specification are incorporated herein by reference in their entirety.

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

## Claims

1. A method for performing a bioproduction process comprising the production of a transformed cell population, the method comprising the step of culturing a cell population at least partially embedded in a hydrogel matrix forming a three-dimensional structure, wherein the hydrogel matrix also comprises a transforming agent, and wherein the growth of the cell population at least partially embedded in the hydrogel matrix results in the transformation of at least a part of the cell population by uptake of the transforming agent.

2. The method of claim 1, wherein the hydrogel structure has been obtained by depositing one or more bioink compositions each comprising one or more biomaterials capable of forming a hydrogel and a cell population and/or a transforming agent in a controlled three-dimensional shape to obtain a cellularised structure, wherein at least one of the bioink compositions comprises a cell population and at least one of the bioink compositions comprises a transforming agent, optionally wherein the bioink composition comprises a liquid culture medium and/or wherein the bioink composition has been obtained by mixing one or more solutions each comprising biomaterials capable of forming a hydrogel and/or cells in a liquid culture medium.

3. The method of any preceding claim, wherein the hydrogel structure has been obtained by depositing a bioink composition comprising one or more biomaterials capable of forming a hydrogel, a cell population and a transforming agent.

4. The method of any preceding claim, wherein the hydrogel structure comprises a first region comprising the cell population at least partially embedded in the hydrogel matrix and a second region comprising the transforming agent in the hydrogel matrix, optionally wherein the first region comprises a plurality of regions and/or the second region comprises a plurality of regions,

5. The method of any preceding claim, wherein the hydrogel structure has been obtained by depositing one or more first bioink composition(s) comprising one or more biomaterials capable of forming a hydrogel and a cell population to form one or more first regions of the hydrogel structure, and one or more second bioink composition(s) comprising one or more biomaterials capable of forming a hydrogel and a transforming agent to form one or more second regions of the hydrogel structure, optionally wherein the method comprises depositing one or more layers of one or more first bioink composition(s) and depositing one or more layers of one or more second bioink compositions; or wherein the method comprises simultaneously depositing one or more first bioink composition(s) and one or more second bioink composition(s).

6. The method of any preceding claim, wherein the transforming agent comprises a non-viral vector, a viral vector or virus, optionally wherein the non-viral vector is a plasmid.

7. The method of any preceding claim, wherein the transforming agent and/or the hydrogel comprises one or more additives, optionally wherein the one or more additives are selected from a transfection reagent, and a reagent that binds or stabilise viral particles;

and/or wherein the hydrogel has been obtained by consolidating one or more biomaterials capable of forming a hydrogel, wherein at least one of the biomaterials has been chemically modified to improve the stability and/or bioavailability of the transforming agent, optionally wherein the chemical modification comprises the addition of positively charged groups.

8. The method of any preceding claim, wherein the cell population comprises or consists of cells from one or more cell lines, one or more populations of primary cells, or one or more production cell populations, optionally wherein the cell population is a population of mammalian cells, and/or wherein the one or more production cell populations are production cell lines selected from: an MDCK cell line, AGE.CR1™, PRE.C6, a VERO cell line, EB.14, EP.66™, HEK293, BHK21, a CHO cell line, NS0, Sp2, Sf9, SF21, MRC-5, WI-38, a CEF cell line, and a hybridoma cell line, optionally wherein the one or more production cell lines are selected from a VERO cell line, a CHO cell line, an MDCK cell line, and HEK293.

9. The method of any preceding claim, wherein the three-dimensional structure has been obtained by additive manufacturing, optionally wherein the method comprises obtaining a three-dimensional structure comprising a hydrogel matrix by additive manufacturing; and/or wherein obtaining a three-dimensional structure by additive manufacturing comprises depositing a composition at a rate below 0.2 mm³/s; and/or

wherein obtaining a three-dimensional structure by additive manufacturing comprises depositing a composition as a filament, optionally wherein the filament has a diameter between 200 and 800 $\mu$m.

10. The method of any of claims 2 to 9, wherein the method comprises providing the bioink composition(s), depositing the bioink composition(s) in a controlled three-dimensional shape and/or consolidating the deposited bioink composition(s),

optionally wherein consolidating the bioink comprises cross-linking one or more of the biomaterials capable of forming a hydrogel, optionally wherein the cross-linking conditions are set to result in a degree of cross-linking that is compatible with the growth of the cells within the cross-linked matrix and/or wherein consolidating the bioink composition(s) comprises exposing the deposited bioink composition(s) to one or more solutions that crosslink one or more of the biomaterials capable of forming a hydrogel; and/or

wherein providing the bioink composition comprises incubating the bioink composition(s) at a predetermined temperature for a predetermined period of time such that the bioink composition reaches a viscosity compatible with printing without loss of cell viability.

11. The method of any preceding claim, wherein the hydrogel matrix comprises alginate and fibrin, and/or wherein the hydrogel matrix has been obtained by consolidation of a composition comprising alginate, fibrinogen and gelatin, optionally wherein the composition comprises between 1.75% and 26% w/v of gelatin, between 0.15% and 4.2% w/v of alginate, and between 0.15% and 5.25% w/v of fibrinogen and/or wherein consolidation of the composition comprises exposing the composition to a calcium salt and thrombin and/or wherein the composition is deposited in conditions enabling the gelatin in the composition to solidify thereby at least temporarily maintaining the 3D structure of the deposited composition, and/or wherein the cell population and hydrogel matrix structure together form a cellularised structure and culturing the cell population at least partially embedded in a hydrogel matrix forming a three-dimensional structure comprises maintaining the cellularised structure in a liquid culture medium in a bioreactor, preferably wherein the cellularised structure is completely immersed in the culture medium.

12. The method of any preceding claim, wherein the method comprises culturing the cell population for at least 7 days, at least 10 days, at least 15 days, at least 20 days, or up to 30 days and/or wherein the growth of the cell population at least partially embedded in the hydrogel matrix results in the transformation of at least a part of the cell population by uptake of the transforming agent after a predetermined amount of time in culture, after at least 3 days in culture, after at least 4 days in culture, after at least 5 days in culture, after at least 6 days in culture, from 3 days in culture to the end of the culture, or from 6 days in culture to the end of the culture, and/or wherein the method comprises harvesting a cellular product wherein the cellular product comprises a cellular product exclusively or more effectively produced by transformed cells, optionally wherein the step of harvesting the cellular product is performed one or more times during the culturing step and/or at the end of the culturing step, and/or wherein the cellular product is selected from: a virus produced by transformed cells, an expression product of a transgene encoded by the transforming agent, a compound produced by the transformed cells, a tissue comprising transformed cells, a cellular popula-

**EP 4 141 096 A1**

tion comprising transformed cells.

13. A bioink composition comprising one or more biomaterials capable of forming a hydrogel, a cell population and a transforming agent.

14. A cellularised structure comprising a hydrogel matrix forming a three-dimensional structure, a cell population at least partially embedded in the hydrogel matrix, and a transforming agent, wherein the cellularised structure has been obtained by depositing one or more bioink compositions each comprising one or more biomaterials capable of forming a hydrogel and a cell population and/or a transforming agent in a controlled three-dimensional shape, wherein at least one of the bioink compositions comprises a cell population and at least one of the bioink compositions comprises a transforming agent.

15. A system for performing a bioproduction process comprising the production of a transformed cell population, the system comprising:

a bioreactor and
one or more cellularised structure(s) according to claim 14.

Fig. 1

Fig. 2A

PROVIDE BIO-INK COMPOSITION(S) — 220

DEPOSIT BIO-INK COMPOSITION — 230

CONSOLIDATE BIO-INK COMPOSITION — 240

CULTURE CELL POPULATION IN 3D HYDROGEL STRUCTURE

(TRANFORMATION IN HYDROGEL TRIGGERED BY CELL GROWTH) — 250

HARVEST CELLULAR PRODUCT OF TRANSFORMED CELLS — 260

Fig. 2B

Fig. 3

Fig. 4

Fig. 5

EP 4 141 096 A1

Fig. 6

Fig. 7

Fig. 8

DNA labelling + bioink + Cacl2 + TAG + Thromb 4x

DNA labelling + bioink + Cacl2 + TAG 4x

DNA labelling + bioink + Cacl2 + Thromb 4x

DNA labelling + bioink + Cacl2 4x

Fig. 9

Fig. 10

Fig. 11

Fig. 12

Biomaterial cellularized

Biomaterial additionned
of transforming agents

Fig. 13

Fig. 14

Fig. 15A

Fig. 15B

Europäisches
Patentamt

European
Patent Office

Office européen
des brevets

# EUROPEAN SEARCH REPORT

Application Number

EP 21 30 6144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/190162 A1 (LEE MOO-YEAL [US] ET AL) 4 August 2011 (2011-08-04) * paragraph [0059]; figure 4 * ----- | 1-15 | INV. C12M1/12 B33Y70/00 C12M1/26 |
| X | GONZALEZ-FERNANDEZ T ET AL: "Pore-forming bioinks to enable spatio-temporally defined gene delivery in bioprinted tissues", JOURNAL OF CONTROLLED RELEASE, vol. 301, 8 March 2019 (2019-03-08), pages 13-27, XP085672782, ISSN: 0168-3659, DOI: 10.1016/J.JCONREL.2019.03.006 * Sections 3.2, 3.4, 3.5; figure 3d * ----- | 1-15 | |

TECHNICAL FIELDS
SEARCHED      (IPC)

C12M
B33Y

The present search report has been drawn up for all claims

| Place of search | Date of completion of the search | Examiner |
|---|---|---|
| Berlin | 11 February 2022 | Pantelidis, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 30 6144

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2022

| Patent document cited in search report | Publication date | Patent family member(s) | Publication date |
|---|---|---|---|
| US 2011190162 A1 | 04-08-2011 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190002836 A

- US 2019002836 A1

**Non-patent literature cited in the description**

- **MELLOT et al.** *Ann Biomed Eng,* March 2013, vol. 41 (3), 446-468
- **PADMANABHAN ; SMITH.** *Pharm Dev Technol,* January 2002, vol. 7 (1), 97-101
- **POURCHET et al.** *Adv. Healthcare Mater.,* 2017, 1601101
- **POURCHET et al.** *Bioprinting,* December 2020, vol. 20, e00114

- **SAKHR et al.** *Int J Mol Sci,* April 2021, vol. 22 (7), 3290
- **THAKOR et al.** *Mol. Ther.,* 2007, vol. 15 (12), 2124-2131
- **SAPET et al.** *Ther. Deliv.,* 2013, vol. 4 (6), 673-685